# EUROPEAN PATENT APPLICATION

(11) **EP 2 290 107 A1**
(43) Date of publication of application: **02.03.2011**
(21) Application number: 10012620.0
(22) Date of filing: 07.01.2004
(51) Int. Cl.: C12Q 1/70, C12Q 1/68, G01N 33/53, C12N 15/10

(54) **Kunitz domain library**

(30) Priority: 07.01.2003 US 438491 P
(62) Divisional of application: 04700617.6
(71) Applicant: Dyax Corporation, Cambridge, MA 02139 (US)
(72) Inventor: Ladner, Robert Charles, Ljamsville, MD 21754 (US); Nixon, Andrew, Hanover, MA 02339 (US)
(74) Representative: Wichmann, Hendrik

(57) **Abstract**

Disclosed are libraries, vectors, phage particles, host cells, and methods for displaying a Kunitz domain. The libraries can include Kunitz domains that vary in at least two interaction loops with respect to one another. Varied Kunitz domains can be displayed on phage at a low valency.

SEQUENCE LISTING

<110> Dyax Corporation

<120> KUNITZ DOMAIN LIBRARY

<130> F63683PCEPT1

<140> Divisional of EP 04700617.6
<141> 2004-01-07

<150> US 60/438,491
<151> 2003-01-07

<160> 12

<170> FastSEQ for Windows Version 4.0

<210> 1
<211> 58
<212> PRT
<213> Artificial Sequence

<220>
<223> Kunitz domain 1 of LACI

<400> 1
Met His Ser Phe Cys Ala Phe Lys Ala Asp Asp Gly Pro Cys Lys Ala
 1               5                  10                  15
Ile Met Lys Arg Phe Phe Phe Asn Ile Phe Thr Arg Gln Cys Glu Glu
            20                  25                  30
Phe Ile Tyr Gly Gly Cys Glu Gly Asn Gln Asn Arg Phe Glu Ser Leu
        35                  40                  45
Glu Glu Cys Lys Lys Met Cys Thr Arg Asp
    50                  55

<210> 2
<211> 64
<212> PRT
<213> Artificial Sequence

<220>
<223> Exemplary Kunitz domain

<220>
<221> VARIANT
<222> 1-4
<223> Xaa = any amino acid, or absent

<220>
<221> VARIANT
<222> 6-8, 10-14
<223> Xaa = any amino acid but preferably not Cys

<220>
<221> VARIANT
<222> 9
<223> Xaa = any amino acid but preferably not Cys,
      or absent

<220>
<221> VARIANT
<222> 16-30
<223> Xaa = any amino acid but preferably not Cys

<220>

<221> VARIANT
<222> 31-33
<223> Xaa = any amino acid, or absent

<220>
<221> VARIANT
<222> 35-41
<223> Xaa = any amino acid but preferably not Cys

<220>
<221> VARIANT
<222> (43)...(56)
<223> Xaa = any amino acid but preferably not Cys

<220>
<221> VARIANT
<222> (58)...(60)
<223> Xaa = any amino acid but preferably not Cys

<220>
<221> VARIANT
<222> (62)...(64)
<223> Xaa = any amino acid, or absent

<400> 2
Xaa Xaa Xaa Xaa Cys Xaa Xaa Xaa Xaa Xaa Xaa Xaa Xaa Xaa Cys Xaa
 1               5                  10                  15
Xaa Xaa Xaa Xaa Xaa Xaa Xaa Xaa Xaa Xaa Xaa Xaa Xaa Xaa Xaa Xaa
            20                  25                  30
Xaa Cys Xaa Xaa Xaa Xaa Xaa Xaa Xaa Cys Xaa Xaa Xaa Xaa Xaa Xaa
        35                  40                  45
Xaa Xaa Xaa Xaa Xaa Xaa Xaa Xaa Cys Xaa Xaa Xaa Cys Xaa Xaa Xaa
    50                  55                  60

<210> 3
<211> 304
<212> PRT
<213> Homo sapiens

<400> 3
Met Ile Tyr Thr Met Lys Lys Val His Ala Leu Trp Ala Ser Val Cys
 1               5                  10                  15
Leu Leu Leu Asn Leu Ala Pro Ala Pro Leu Asn Ala Asp Ser Glu Glu
            20                  25                  30
Asp Glu Glu His Thr Ile Ile Thr Asp Thr Glu Leu Pro Pro Leu Lys
        35                  40                  45
Leu Met His Ser Phe Cys Ala Phe Lys Ala Asp Asp Gly Pro Cys Lys
    50                  55                  60
Ala Ile Met Lys Arg Phe Phe Phe Asn Ile Phe Thr Arg Gln Cys Glu
65                  70                  75                  80
Glu Phe Ile Tyr Gly Gly Cys Glu Gly Asn Gln Asn Arg Phe Glu Ser
                85                  90                  95
Leu Glu Glu Cys Lys Lys Met Cys Thr Arg Asp Asn Ala Asn Arg Ile
            100                 105                 110
Ile Lys Thr Thr Leu Gln Gln Glu Lys Pro Asp Phe Cys Phe Leu Glu
        115                 120                 125
Glu Asp Pro Gly Ile Cys Arg Gly Tyr Ile Thr Arg Tyr Phe Tyr Asn
    130                 135                 140
Asn Gln Thr Lys Gln Cys Glu Arg Phe Lys Tyr Gly Gly Cys Leu Gly
145                 150                 155                 160
Asn Met Asn Asn Phe Glu Thr Leu Glu Glu Cys Lys Asn Ile Cys Glu
                165                 170                 175
Asp Gly Pro Asn Gly Phe Gln Val Asp Asn Tyr Gly Thr Gln Leu Asn
            180                 185                 190
Ala Val Asn Asn Ser Leu Thr Pro Gln Ser Thr Lys Val Pro Ser Leu
        195                 200                 205
Phe Glu Phe His Gly Pro Ser Trp Cys Leu Thr Pro Ala Asp Arg Gly
    210                 215                 220

Leu Cys Arg Ala Asn Glu Asn Arg Phe Tyr Tyr Asn Ser Val Ile Gly
225                 230                 235                 240
Lys Cys Arg Pro Phe Lys Tyr Ser Gly Cys Gly Gly Asn Glu Asn Asn
                245                 250                 255
Phe Thr Ser Lys Gln Glu Cys Leu Arg Ala Cys Lys Lys Gly Phe Ile
            260                 265                 270
Gln Arg Ile Ser Lys Gly Gly Leu Ile Lys Thr Lys Arg Lys Arg Lys
        275                 280                 285
Lys Gln Arg Val Lys Ile Ala Tyr Glu Glu Ile Phe Val Lys Asn Met
    290                 295                 300

<210> 4
<211> 58
<212> PRT
<213> Artificial Sequence

<220>
<223> First exemplary Kunitz domain library

<220>
<221> VARIANT
<222> 15, 17, 18
<223> Xaa = Ala,  Asp, Glu, Phe, Gly, His, Ile, Lys, Leu,
      Met, Asn, Gln, Arg, Ser, Thr, Val, Trp, Tyr , (no
      Cys or Pro)

<220>
<221> VARIANT
<222> 11,13, 19, 34, 39
<223> Xaa = Ala,  Asp, Glu, Phe, Gly, His, Ile, Lys,
      Leu, Met, Asn, Pro, Gln, Arg, Ser, Thr, Val, Trp,
      Tyr , no Cys

<220>
<221> VARIANT
<222> 16
<223> Xaa = Ala, Gly, Glu, Asp, His, Thr

<220>
<221> VARIANT
<222> 40
<223> Xaa = Ala or Gly

<400> 4
Met His Ser Phe Cys Ala Phe Lys Ala Asp Xaa Gly Xaa Cys Xaa Xaa
 1               5                  10                  15
Xaa Xaa Xaa Arg Phe Phe Phe Asn Ile Phe Thr Arg Gln Cys Glu Glu
            20                  25                  30
Phe Xaa Tyr Gly Gly Cys Xaa Xaa Asn Gln Asn Arg Phe Glu Ser Leu
        35                  40                  45
Glu Glu Cys Lys Lys Met Cys Thr Arg Asp
    50                  55

<210> 5
<211> 174
<212> DNA
<213> Artificial Sequence

<220>
<223> First exemplary Kunitz domain library

<220>
<221> CDS
<222> (1)...(174)

<220>
<221> misc_feature
<222> 31-33, 37-39, 43-57, 100-102, 115-120

<223> n = a, g, c, or t

<400> 5
atg cat tcc ttc tgc gcc ttc aag gcc gac nnn ggt nnn tgt nnn nnn      48
Met His Ser Phe Cys Ala Phe Lys Ala Asp Xaa Gly Xaa Cys Xaa Xaa
 1               5                  10                  15

nnn nnn nnn cgt ttc ttc ttc aac atc ttc acg cgt cag tgc gaa gag      96
Xaa Xaa Xaa Arg Phe Phe Phe Asn Ile Phe Thr Arg Gln Cys Glu Glu
            20                  25                  30

ttc nnn tac ggt ggt tgt nnn nnn aac cag aac cgg ttc gaa tct cta      144
Phe Xaa Tyr Gly Gly Cys Xaa Xaa Asn Gln Asn Arg Phe Glu Ser Leu
        35                  40                  45

gag gaa tgt aag aag atg tgc act cgt gac                              174
Glu Glu Cys Lys Lys Met Cys Thr Arg Asp
    50                  55


<210> 6
<211> 58
<212> PRT
<213> Artificial Sequence

<220>
<223> Second exemplary Kunitz domain library

<220>
<221> VARIANT
<222> 15, 17, 18, 40, 46
<223> Xaa = Ala,  Asp, Glu, Phe, Gly, His, Ile, Lys, Leu,
      Met, Asn, Gln, Arg, Ser, Thr, Val, Trp, Tyr , (no
      Cys or Pro)

<220>
<221> VARIANT
<222> 11, 13, 19, 32, 34, 39
<223> Xaa = Ala,  Asp, Glu, Phe, Gly, His, Ile, Lys,
      Leu, Met, Asn, Pro, Gln, Arg, Ser, Thr, Val, Trp,
      Tyr , (no Cys)

<220>
<221> VARIANT
<222> 16
<223> Xaa = Ala, Gly, Glu, Asp, His, Thr

<400> 6
Met His Ser Phe Cys Ala Phe Lys Ala Asp Xaa Gly Xaa Cys Xaa Xaa
 1               5                  10                  15
Xaa Xaa Xaa Arg Phe Phe Phe Asn Ile Phe Thr Arg Gln Cys Glu Xaa
            20                  25                  30
Phe Xaa Tyr Gly Gly Cys Xaa Xaa Asn Gln Asn Arg Phe Xaa Ser Leu
        35                  40                  45
Glu Glu Cys Lys Lys Met Cys Thr Arg Asp
    50                  55

<210> 7
<211> 174
<212> DNA
<213> Artificial Sequence

<220>
<223> Second exemplary Kunitz domain library

<220>
<221> CDS
<222> (1)...(174)

<220>
<221> misc_feature
<222> 31-33, 37-39, 43-57, 94-96, 100-102, 115-120, 136-138
<223> n = a, g, c, or t

<400> 7
atg cat tcc ttc tgc gcc ttc aag gcc gac nnn ggt nnn tgt nnn nnn 48
Met His Ser Phe Cys Ala Phe Lys Ala Asp Xaa Gly Xaa Cys Xaa Xaa
1               5                   10                  15

nnn nnn nnn cgt ttc ttc ttc aac atc ttc acg cgt cag tgc gag nnn 96
Xaa Xaa Xaa Arg Phe Phe Phe Asn Ile Phe Thr Arg Gln Cys Glu Xaa
            20                  25                  30

ttc nnn tac ggt ggt tgt nnn nnn aac cag aac cgg ttc nnn tct cta 144
Phe Xaa Tyr Gly Gly Cys Xaa Xaa Asn Gln Asn Arg Phe Xaa Ser Leu
        35                  40                  45

gag gaa tgt aag aag atg tgc act cgt gac 174
Glu Glu Cys Lys Lys Met Cys Thr Arg Asp
    50                  55


<210> 8
<211> 235
<212> PRT
<213> Artificial Sequence

<220>
<223> Varied display protein

<400> 8
Met Lys Lys Leu Leu Phe Ala Ile Pro Leu Val Val Pro Phe Tyr Ser
1               5                   10                  15
Met Ala Ala Glu Met His Ser Phe Cys Ala Phe Lys Ala Asp Asp Gly
            20                  25                  30
Pro Cys Lys Ala Ile Met Lys Arg Phe Phe Phe Asn Ile Phe Thr Arg
        35                  40                  45
Gln Cys Glu Glu Phe Ile Tyr Gly Gly Cys Glu Gly Asn Gln Asn Arg
    50                  55                  60
Phe Glu Ser Leu Glu Glu Cys Lys Lys Met Cys Thr Arg Asp Ser Ala
65                  70                  75                  80
Ser Ser Ala Ser Gly Asp Phe Asp Tyr Glu Lys Met Ala Asn Ala Asn
                85                  90                  95
Lys Gly Ala Met Thr Glu Asn Ala Asp Glu Asn Ala Leu Gln Ser Asp
            100                 105                 110
Ala Lys Gly Lys Leu Asp Ser Val Ala Thr Asp Tyr Gly Ala Ala Ile
        115                 120                 125
Asp Gly Phe Ile Gly Asp Val Ser Gly Leu Ala Asn Gly Asn Gly Ala
    130                 135                 140
Thr Gly Asp Phe Ala Gly Ser Asn Ser Gln Met Ala Gln Val Gly Asp
145                 150                 155                 160
Gly Asp Asn Ser Pro Leu Met Asn Asn Phe Arg Gln Tyr Leu Pro Ser
                165                 170                 175
Leu Pro Gln Ser Val Glu Cys Arg Pro Phe Val Phe Gly Ala Gly Lys
            180                 185                 190
Pro Tyr Glu Phe Ser Ile Asp Cys Asp Lys Ile Asn Leu Phe Arg Gly
        195                 200                 205
Val Phe Ala Phe Leu Leu Tyr Val Ala Thr Phe Met Tyr Val Phe Ser
    210                 215                 220
Thr Phe Ala Asn Ile Leu Arg Asn Lys Glu Ser
225                 230                 235

<210> 9
<211> 940
<212> DNA
<213> Artificial Sequence

<220>
<223> Exemplary  phage vector DY3P82_LACIK1

<400> 9
cgcaacgcaa ttaatgtgag ttagctcact cattaggcac cccaggcttt acactttatg      60
cttccggctc gtatgttgtg tggaattgtg agcggataac aatttcacac aggaaacagc     120
tatgaccatg attacgccaa gctttggagc cttttttttg gagattttca acatgaaaaa     180
attattattc gcaattcctt tagttgttcc tttctattcc atggcggccg agatgcattc     240
attctgcgct ttcaaagctg atgacggtcc gtgtaaagct atcatgaaac gtttcttctt     300
caacattttc acgcgtcaat gtgaagagtt catttacggt ggttgtgaag gtaaccagaa     360
ccggttcgaa tctctagagg aatgtaagaa gatgtgcact cgtgattctg ctagctctgc     420
tagtggcgac ttcgactacg agaaaatggc taatgccaac aaaggcgcca tgactgagaa     480
cgctgacgag aatgctttgc aaagcgatgc caagggtaag ttagacagcg tcgcgaccga     540
ctatggcgcc gccatcgacg gctttatcgg cgatgtcagt ggtttggcca acggcaacgg     600
agccaccgga gacttcgcag gttcgaattc tcagatggcc caggttggag atggggacaa     660
cagtccgctt atgaacaact ttagacagta ccttccgtct cttccgcaga gtgtcgagtg     720
ccgtccattc gttttcggtg ccggcaagcc ttacgagttc agcatcgact gcgataagat     780
caatcttttc cgcggcgttt tcgctttctt gctatacgtc gctactttca tgtacgtttt     840
cagcactttc gccaatattt tacgcaacaa agaaagctag tgatctccta ggaagcccgc     900
ctaatgagcg ggcttttttt ttctggtatg catcctgagg                           940

<210> 10
<211> 60
<212> PRT
<213> Artificial Sequence

<220>
<223> Exemplary Kunitz domain

<220>
<221> VARIANT
<222> 11, 13, 19
<223> Xaa = Ala, Asp, Glu, Phe, Gly, His, Ile, Lys, Leu,
      Met,Asn, Pro, Gln,Arg, Ser, Thr, Val, Trp or Tyr

<220>
<221> VARIANT
<222> 15, 17, 18
<223> Xaa = Ala, Asp, Glu, Phe, Gly, His, Ile, Lys, Leu,
      Met,Asn,  Gln,Arg, Ser, Thr, Val, Trp or Tyr

<220>
<221> VARIANT
<222> 16
<223> Xaa = Ala, Gly, Glu, Asp, His or Thr

<220>
<221> VARIANT
<222> 34, 39
<223> Xaa = Ala, Cys, Asp, Glu, Phe, Gly, His, Ile, Lys,
      Leu, Met, Asn, Pro, Gln, Arg, Ser, Thr, Val, Trp
      or Tyr

<220>
<221> VARIANT
<222> 40
<223> Xaa = Gly or Ala

<400> 10
Met His Ser Phe Cys Ala Phe Lys Ala Asp Xaa Gly Xaa Cys Xaa Xaa
1               5                   10                  15
Xaa Xaa Xaa Arg Phe Phe Phe Asn Ile Phe Thr Arg Gln Cys Glu Glu
            20                  25                  30
Phe Xaa Tyr Gly Gly Cys Xaa Xaa Asn Gln Asn Arg Phe Glu Ser Leu
        35                  40                  45
Glu Glu Cys Lys Lys Met Cys Thr Arg Asp Gly Ala
    50                  55                  60

<210> 11
<211> 8829
<212> DNA
<213> Artificial Sequence

<220>
<223> Exemplary vector sequence

<400> 11
aatgctacta ctattagtag aattgatgcc accttttcag ctcgcgcccc aaatgaaaat 60
atagctaaac aggttattga ccatttgcga aatgtatcta atggtcaaac taaatctact 120
cgttcgcaga attgggaatc aactgttata tggaatgaaa cttccagaca ccgtacttta 180
gttgcatatt taaaacatgt tgagctacag cattatattc agcaattaag ctctaagcca 240
tccgcaaaaa tgacctctta tcaaaaggag caattaaagg tactctctaa tcctgacctg 300
ttggagtttg cttccggtct ggttcgcttt gaagctcgaa ttaaaacgcg atatttgaag 360
tctttcgggc ttcctcttaa tctttttgat gcaatccgct ttgcttctga ctataatagt 420
cagggtaaag acctgatttt tgatttatgg tcattctcgt tttctgaact gtttaaagca 480
tttgaggggg attcaatgaa tatttatgac gattccgcag tattggacgc tatccagtct 540
aaacatttta ctattacccc ctctggcaaa acttcttttg caaaagcctc tcgctatttt 600
ggtttttatc gtcgtctggt aaacgagggt tatgatagtg ttgctcttac tatgcctcgt 660
aattcctttt ggcgttatgt atctgcatta gttgaatgtg gtattcctaa atctcaactg 720
atgaatcttt ctacctgtaa taatgttgtt ccgttagttc gttttattaa cgtagatttt 780
tcttcccaac gtcctgactg gtataatgag ccagttctta aaatcgcata aggtaattca 840
caatgattaa agttgaaatt aaaccatctc aagcccaatt tactactcgt tctggtgttt 900
ctcgtcaggg caagccttat tcactgaatg agcagctttg ttacgttgat ttgggtaatg 960
aatatccggt tcttgtcaag attactcttg atgaaggtca gccagcctat gcgcctggtc 1020
tgtacaccgt tcatctgtcc tctttcaaag ttggtcagtt cggttccctt atgattgacc 1080
gtctgcgcct cgttccggct aagtaacatg gagcaggtcg cggatttcga cacaatttat 1140
caggcgatga tacaaatctc cgttgtactt tgtttcgcgc ttggtataat cgctgggggt 1200
caaagatgag tgttttagtg tattcttttg cctctttcgt tttaggttgg tgccttcgta 1260
gtggcattac gtattttacc cgtttaatgg aaacttcctc atgaaaaagt ctttagtcct 1320
caaagcctct gtagccgttg ctaccctcgt tccgatgctg tctttcgctg ctgagggtga 1380
cgatcccgca aaagcggcct ttaactccct gcaagcctca gcgaccgaat atatcggtta 1440
tgcgtgggcg atggttgttg tcattgtcgg cgcaactatc ggtatcaagc tgtttaagaa 1500
attcacctcg aaagcaagct gataaaccga tacaattaaa ggctcctttt ggagcctttt 1560
ttttggaga ttttcaacgt gaaaaaatta ttattcgcaa ttcctttagt tgttcctttc 1620
tattctcact ccgctgaaac tgttgaaagt tgtttagcaa aatcccatac agaaaattca 1680
tttactaacg tctggaaaga cgacaaaact ttagatcgtt acgctaacta tgagggctgt 1740
ctgtggaatg ctacaggcgt tgtagtttgt actggtgacg aaactcagtg ttacggtaca 1800
tgggttccta ttgggcttgc tatccctgaa aatgagggtg gtggctctga gggtggcggt 1860
tctgagggtg gcggttctga gggtggcggt actaaacctc ctgagtacgg tgatacacct 1920
attccgggct atacttatat caaccctctc gacggcactt atccgcctgg tactgagcaa 1980
aaccccgcta atcctaatcc ttctcttgag gagtctcagc ctcttaatac tttcatgttt 2040
cagaataata ggttccgaaa taggcagggg gcattaactg tttatacggg cactgttact 2100
caaggcactg accccgttaa aacttattac cagtacactc ctgtatcatc aaaagccatg 2160
tatgacgctt actggaacgg taaattcaga gactgcgctt tccattctgg ctttaatgag 2220
gatttatttg tttgtgaata tcaaggccaa tcgtctgacc tgcctcaacc tcctgtcaat 2280
gctggcggcg gctctggtgg tggttctggt ggcggctctg agggtggtgg ctctgagggt 2340
ggcggttctg agggtggcgg ctctgaggga ggcggttccg gtggtggctc tggttccggt 2400
gattttgatt atgaaaagat ggcaaacgct aataaggggg ctatgaccga aaatgccgat 2460
gaaaacgcgc tacagtctga cgctaaaggc aaacttgatt ctgtcgctac tgattacggt 2520
gctgctatcg atggtttcat tggtgacgtt tccggccttg ctaatggtaa tggtgctact 2580
ggtgattttg ctggctctaa ttcccaaatg gctcaagtcg gtgacggtga taattcacct 2640
ttaatgaata atttccgtca atatttacct tccctccctc aatcggttga atgtcgccct 2700
tttgtctttg gcgctggtaa accatatgaa ttttctattg attgtgacaa aataaactta 2760
ttccgtggtg tctttgcgtt tcttttatat gttgccacct ttatgtatgt attttctacg 2820
tttgctaaca tactgcgtaa taaggagtct taatcatgcc agttcttttg ggtattccgt 2880
tattattgcg tttcctcggt ttccttctgg taactttgtt cggctatctg cttacttttc 2940
ttaaaaaggg cttcggtaag atagctattg ctatttcatt gtttcttgct cttattattg 3000
ggcttaactc aattcttgtg ggttatctct ctgatattag cgctcaatta ccctctgact 3060
ttgttcaggg tgttcagtta attctcccgt ctaatgcgct tccctgtttt tatgttattc 3120
tctctgtaaa ggctgctatt ttcatttttg acgttaaaca aaaaatcgtt tcttatttgg 3180
attgggataa ataatatggc tgtttatttt gtaactggca aattaggctc tggaaagacg 3240
ctcgttagcg ttggtaagat tcaggataaa attgtagctg ggtgcaaaat agcaactaat 3300
cttgatttaa ggcttcaaaa cctcccgcaa gtcgggaggt tcgctaaaac gcctcgcgtt 3360
cttagaatac cggataagcc ttctatatct gatttgcttg ctattgggcg cggtaatgat 3420
tcctacgatg aaaataaaaa cggcttgctt gttctcgatg agtgcggtac ttggtttaat 3480

acccgttctt ggaatgataa ggaaagacag ccgattattg attggtttct acatgctcgt 3540
aaattaggat gggatattat ttttcttgtt caggacttat ctattgttga taaacaggcg 3600
cgttctgcat tagctgaaca tgttgtttat tgtcgtcgtc tggacagaat tactttacct 3660
tttgtcggta ctttatattc tcttattact ggctcgaaaa tgcctctgcc taaattacat 3720
gttggcgttg ttaaatatgg cgattctcaa ttaagcccta ctgttgagcg ttggctttat 3780
actggtaaga atttgtataa cgcatatgat actaaacagg ctttttctag taattatgat 3840
tccggtgttt attcttattt aacgccttat ttatcacacg gtcggtattt caaaccatta 3900
aatttaggtc agaagatgaa attaactaaa atatatttga aaaagttttc tcgcgttctt 3960
tgtcttgcga ttggatttgc atcagcattt acatatagtt atataaccca acctaagccg 4020
gaggttaaaa aggtagtctc tcagacctat gattttgata aattcactat tgactcttct 4080
cagcgtctta atctaagcta tcgctatgtt ttcaaggatt ctaagggaaa attaattaat 4140
agcgacgatt tacagaagca aggttattca ctcacatata ttgatttatg tactgtttcc 4200
attaaaaaag gtaattcaaa tgaaattgtt aaatgtaatt aattttgttt tcttgatgtt 4260
tgtttcatca tcttcttttg ctcaggtaat tgaaatgaat aattcgcctc tgcgcgattt 4320
tgtaacttgg tattcaaagc aatcaggcga atccgttatt gtttctcccg atgtaaaagg 4380
tactgttact gtatattcat ctgacgttaa acctgaaaat ctacgcaatt tctttatttc 4440
tgttttacgt gcaaataatt ttgatatggt aggttctaac ccttccataa ttcagaagta 4500
taatccaaac aatcaggatt atattgatga attgccatca tctgataatc aggaatatga 4560
tgataattcc gctccttctg gtggtttctt tgttccgcaa aatgataatg ttactcaaac 4620
ttttaaaatt aataacgttc gggcaaagga tttaatacga gttgtcgaat tgtttgtaaa 4680
gtctaatact tctaaatcct caaatgtatt atctattgac ggctctaatc tattagttgt 4740
tagtgctcct aaagatattt tagataacct tcctcaattc ctttcaactg ttgatttgcc 4800
aactgaccag atattgattg agggtttgat atttgaggtt cagcaaggtg atgctttaga 4860
tttttcattt gctgctggct ctcagcgtgg cactgttgca ggcggtgtta atactgaccg 4920
cctcacctct gttttatctt ctgctggtgg ttcgttcggt atttttaatg gcgatgtttt 4980
agggctatca gttcgcgcat taaagactaa tagccattca aaaatattgt ctgtgccacg 5040
tattcttacg ctttcaggtc agaagggttc tatctctgtt ggccagaatg tcccttttat 5100
tactggtcgt gtgactggtg aatctgccaa tgtaaataat ccatttcaga cgattgagcg 5160
tcaaaatgta ggtatttcca tgagcgtttt tcctgttgca atggctggcg gtaatattgt 5220
tctggatatt accagcaagg ccgatagttt gagttcttct actcaggcaa gtgatgttat 5280
tactaatcaa agaagtattg ctacaacggt taatttgcgt gatggacaga ctcttttact 5340
cggtggcctc actgattata aaaacacttc tcaggattct ggcgtaccgt tcctgtctaa 5400
aatcccttta atcggcctcc tgtttagctc ccgctctgat tctaacgagg aaagcacgtt 5460
atacgtgctc gtcaaagcaa ccatagtacg cgccctgtag cggcgcatta agcgcggcgg 5520
gtgtggtggt tacgcgcagc gtgaccgcta cacttgccag cgccctagcg cccgctcctt 5580
tcgctttctt cccttccttt ctcgccacgt tcgccggctt tccccgtcaa gctctaaatc 5640
gggggctccc tttagggttc cgatttagtg ctttacggca cctcgacccc aaaaaacttg 5700
atttgggtga tggttggcca tcgccctgat agacggtttt tcgccctttg acgttggagt 5760
ccacgttctt taatagtgga ctcttgttcc aaactggaac aacactcaac cctatctcgg 5820
gctattcttt tgatttataa gggattttgc cgatttcgga accaccatca aacaggattt 5880
tcgcctgctg gggcaaacca gcgtggaccg cttgctgcaa ctctctcagg gccaggcggt 5940
gaagggcaat cagctgttgc ccgtctcact ggtgaaaaga aaaaccaccc tggatccaag 6000
cttgcaggtg gcacttttcg gggaaatgtg cgcggaaccc ctatttgttt atttttctaa 6060
atacattcaa atatgtatcc gctcatgaga caataaccct gataaatgct tcaataatat 6120
tgaaaaagga agagtatgag tattcaacat ttccgtgtcg cccttattcc cttttttgcg 6180
gcattttgcc ttcctgtttt tgctcaccca gaaacgctgg tgaaagtaaa agatgctgaa 6240
gatcagttgg gcgcactagt gggttacatc gaactggatc tcaacagcgg taagatcctt 6300
gagagttttc gccccgaaga acgttttcca atgatgagca cttttaaagt tctgctatgt 6360
ggcgcggtat tatcccgtat tgacgccggg caagagcaac tcggtcgccg catacactat 6420
tctcagaatg acttggttga gtactcacca gtcacagaaa agcatcttac ggatggcatg 6480
acagtaagag aattatgcag tgctgccata accatgagtg ataacactgc ggccaactta 6540
cttctgacaa cgatcggagg accgaaggag ctaaccgctt ttttgcacaa catgggggat 6600
catgtaactc gccttgatcg ttgggaaccg gagctgaatg aagccatacc aaacgacgag 6660
cgtgacacca cgatgcctgt agcaatggca acaacgttgc gcaaactatt aactggcgaa 6720
ctacttactc tagcttcccg gcaacaatta atagactgga tggaggcgga taaagttgca 6780
ggaccacttc tgcgctcggc ccttccggct ggctggttta ttgctgataa atctggagcc 6840
ggtgagcgtg ggtctcgcgg tatcattgca gcactggggc cagatggtaa gccctcccgt 6900
atcgtagtta tctacacgac ggggagtcag gcaactatgg atgaacgaaa tagacagatc 6960
gctgagatag gtgcctcact gattaagcat tggtaactgt cagaccaagt ttactcatat 7020
atactttaga ttgatttaaa acttcatttt taatttaaaa ggatctaggt gaagatcctt 7080
tttgataatc tcatgaccaa aatcccttaa cgtgagtttt cgttccactg tacgtaagac 7140
ccccaagctt gtcgactgaa tggcgaatgg cgctttgcct ggtttccggc accagaagcg 7200
gtgccggaaa gctggctgga gtgcgatctt cctgacgctc gagcgcaacg caattaatgt 7260
gagttagctc actcattagg caccccaggc tttacacttt atgcttccgg ctcgtatgtt 7320
gtgtggaatt gtgagcggat aacaatttca cacaggaaac agctatgacc atgattacgc 7380
caagctttgg agcctttttt ttggagattt tcaacgtgaa aaaattatta ttcgcaattc 7440
ctttagttgt tcctttctat tccatggcgg ccgagatgca ttcattcatt ttcacgcgtc 7500
agtgcgagga aaaccggttc gagtctctag aggaatgtaa gaagatgtgc actcgtgatt 7560

ctgctagctc tgctagtggc gacttcgact acgagaaaat ggctaatgcc aacaaaggcg 7620
ccatgactga gaacgctgac gagaatgctt tgcaaagcga tgccaagggt aagttagaca 7680
gcgtcgcgac cgactatggc gccgccatcg acggctttat cggcgatgtc agtggtttgg 7740
ccaacggcaa cggagccacc ggagacttcg caggttcgaa ttctcagatg gcccaggttg 7800
gagatgggga caacagtccg cttatgaaca actttagaca gtaccttccg tctcttccgc 7860
agagtgtcga gtgccgtcca ttcgttttcg gtgccggcaa gccttacgag ttcagcatcg 7920
actgcgataa gatcaatctt ttccgcggcg ttttcgcttt cttgctatac gtcgctactt 7980
tcatgtacgt tttcagcact ttcgccaata ttttacgcaa caaagaaagc tagtgatctc 8040
ctaggaagcc cgcctaatga gcgggctttt tttttctggt atgcatcctg aggccgatac 8100
tgtcgtcgtc ccctcaaact ggcagatgca cggttacgat gcgcccatct acaccaacgt 8160
gacctatccc attacggtca atccgccgtt tgttcccacg gagaatccga cgggttgtta 8220
ctcgctcaca tttaatgttg atgaaagctg gctacaggaa ggccagacgc gaattatttt 8280
tgatggcgtt cctattggtt aaaaaatgag ctgatttaac aaaaatttaa tgcgaatttt 8340
aacaaaatat taacgtttac aatttaaata tttgcttata caatcttcct gtttttgggg 8400
cttttctgat tatcaaccgg ggtacatatg attgacatgc tagttttacg attaccgttc 8460
atcgattctc ttgtttgctc cagactctca ggcaatgacc tgatagcctt tgtagatctc 8520
tcaaaaatag ctaccctctc cggcattaat ttatcagcta gaacggttga atatcatatt 8580
gatggtgatt tgactgtctc cggcctttct caccctttg aatctttacc tacacattac 8640
tcaggcattg catttaaaat atatgagggt tctaaaaatt tttatccttg cgttgaaata 8700
aaggcttctc ccgcaaaagt attacagggt cataatgttt ttggtacaac cgatttagct 8760
ttatgctctg aggctttatt gcttaatttt gctaattctt tgccttgcct gtatgattta 8820
ttggatgtt
8829

<210> 12
<211> 8919
<212> DNA
<213> Artificial Sequence

<220>
<223> Exemplary vector sequence

<400> 12
aatgctacta ctattagtag aattgatgcc accttttcag ctcgcgcccc aaatgaaaat 60
atagctaaac aggttattga ccatttgcga aatgtatcta atggtcaaac taaatctact 120
cgttcgcaga attgggaatc aactgttata tggaatgaaa cttccagaca ccgtacttta 180
gttgcatatt taaaacatgt tgagctacag cattatattc agcaattaag ctctaagcca 240
tccgcaaaaa tgacctctta tcaaaaggag caattaaagg tactctctaa tcctgacctg 300
ttggagtttg cttccggtct ggttcgcttt gaagctcgaa ttaaaacgcg atatttgaag 360
tctttcgggc ttcctcttaa tctttttgat gcaatccgct ttgcttctga ctataatagt 420
cagggtaaag acctgatttt tgatttatgg tcattctcgt tttctgaact gtttaaagca 480
tttgaggggg attcaatgaa tatttatgac gattccgcag tattggacgc tatccagtct 540
aaacatttta ctattacccc ctctggcaaa acttcttttg caaaagcctc tcgctatttt 600
ggtttttatc gtcgtctggt aaacgagggt tatgatagtg ttgctcttac tatgcctcgt 660
aattcctttt ggcgttatgt atctgcatta gttgaatgtg gtattcctaa atctcaactg 720
atgaatcttt ctacctgtaa taatgttgtt ccgttagttc gttttattaa cgtagatttt 780
tcttcccaac gtcctgactg gtataatgag ccagttctta aaatcgcata aggtaattca 840
caatgattaa agttgaaatt aaaccatctc aagcccaatt tactactcgt tctggtgttt 900
ctcgtcaggg caagccttat tcactgaatg agcagctttg ttacgttgat ttgggtaatg 960
aatatccggt tcttgtcaag attactcttg atgaaggtca gccagcctat gcgcctggtc 1020
tgtacaccgt tcatctgtcc tctttcaaag ttggtcagtt cggttccctt atgattgacc 1080
gtctgcgcct cgttccggct aagtaacatg gagcaggtcg cggatttcga cacaatttat 1140
caggcgatga tacaaatctc cgttgtactt tgtttcgcgc ttggtataat cgctgggggt 1200
caaagatgag tgttttagtg tattcttttg cctctttcgt tttaggttgg tgccttcgta 1260
gtggcattac gtattttacc cgtttaatgg aaacttcctc atgaaaaagt ctttagtcct 1320
caaagcctct gtagccgttg ctaccctcgt tccgatgctg tctttcgctg ctgagggtga 1380
cgatcccgca aaagcggcct ttaactccct gcaagcctca gcgaccgaat atatcggtta 1440
tgcgtgggcg atggttgttg tcattgtcgg cgcaactatc ggtatcaagc tgtttaagaa 1500
attcacctcg aaagcaagct gataaaccga tacaattaaa ggctcctttt ggagcctttt 1560
tttttggaga ttttcaacgt gaaaaaatta ttattcgcaa ttcctttagt tgttcctttc 1620
tattctcact ccgctgaaac tgttgaaagt tgtttagcaa aatcccatac agaaaattca 1680
tttactaacg tctggaaaga cgacaaaact ttagatcgtt acgctaacta tgagggctgt 1740
ctgtggaatg ctacaggcgt tgtagtttgt actggtgacg aaactcagtg ttacggtaca 1800
tgggttccta ttgggcttgc tatccctgaa aatgagggtg gtggctctga gggtggcggt 1860
tctgagggtg gcggttctga gggtggcggt actaaacctc ctgagtacgg tgatacacct 1920
attccgggct atacttatat caaccctctc gacggcactt atccgcctgg tactgagcaa 1980
aaccccgcta atcctaatcc ttctcttgag gagtctcagc ctcttaatac tttcatgttt 2040

cagaataata ggttccgaaa taggcagggg gcattaactg tttatacggg cactgttact 2100
caaggcactg accccgttaa aacttattac cagtacactc ctgtatcatc aaaagccatg 2160
tatgacgctt actggaacgg taaattcaga gactgcgctt tccattctgg ctttaatgag 2220
gatttatttg tttgtgaata tcaaggccaa tcgtctgacc tgcctcaacc tcctgtcaat 2280
gctggcggcg gctctggtgg tggttctggt ggcggctctg agggtggtgg ctctgagggt 2340
ggcggttctg agggtggcgg ctctgaggga ggcggttccg gtggtggctc tggttccggt 2400
gattttgatt atgaaaagat ggcaaacgct aataaggggg ctatgaccga aaatgccgat 2460
gaaaacgcgc tacagtctga cgctaaaggc aaacttgatt ctgtcgctac tgattacggt 2520
gctgctatcg atggtttcat tggtgacgtt tccggccttg ctaatggtaa tggtgctact 2580
ggtgattttg ctggctctaa ttcccaaatg gctcaagtcg gtgacggtga taattcacct 2640
ttaatgaata atttccgtca atatttacct tccctccctc aatcggttga atgtcgccct 2700
tttgtctttg gcgctggtaa accatatgaa ttttctattg attgtgacaa aataaactta 2760
ttccgtggtg tctttgcgtt tcttttatat gttgccacct ttatgtatgt attttctacg 2820
tttgctaaca tactgcgtaa taaggagtct taatcatgcc agttcttttg ggtattccgt 2880
tattattgcg tttcctcggt ttccttctgg taactttgtt cggctatctg cttacttttc 2940
ttaaaaaggg cttcggtaag atagctattg ctatttcatt gtttcttgct cttattattg 3000
ggcttaactc aattcttgtg ggttatctct ctgatattag cgctcaatta ccctctgact 3060
ttgttcaggg tgttcagtta attctcccgt ctaatgcgct tccctgtttt tatgttattc 3120
tctctgtaaa ggctgctatt ttcatttttg acgttaaaca aaaaatcgtt tcttatttgg 3180
attgggataa ataatatggc tgtttatttt gtaactggca aattaggctc tggaaagacg 3240
ctcgttagcg ttggtaagat tcaggataaa attgtagctg ggtgcaaaat agcaactaat 3300
cttgatttaa ggcttcaaaa cctcccgcaa gtcgggaggt tcgctaaaac gcctcgcgtt 3360
cttagaatac cggataagcc ttctatatct gatttgcttg ctattgggcg cggtaatgat 3420
tcctacgatg aaaataaaaa cggcttgctt gttctcgatg agtgcggtac ttggtttaat 3480
acccgttctt ggaatgataa ggaaagacag ccgattattg attggtttct acatgctcgt 3540
aaattaggat gggatattat ttttcttgtt caggacttat ctattgttga taaacaggcg 3600
cgttctgcat tagctgaaca tgttgtttat tgtcgtcgtc tggacagaat tactttacct 3660
tttgtcggta ctttatattc tcttattact ggctcgaaaa tgcctctgcc taaattacat 3720
gttggcgttg ttaaatatgg cgattctcaa ttaagcccta ctgttgagcg ttggctttat 3780
actggtaaga atttgtataa cgcatatgat actaaacagg ctttttctag taattatgat 3840
tccggtgttt attcttattt aacgccttat ttatcacacg gtcggtattt caaaccatta 3900
aatttaggtc agaagatgaa attaactaaa atatatttga aaaagttttc tcgcgttctt 3960
tgtcttgcga ttggatttgc atcagcattt acatatagtt atataaccca acctaagccg 4020
gaggttaaaa aggtagtctc tcagacctat gattttgata aattcactat tgactcttct 4080
cagcgtctta atctaagcta tcgctatgtt ttcaaggatt ctaagggaaa attaattaat 4140
agcgacgatt tacagaagca aggttattca ctcacatata ttgatttatg tactgtttcc 4200
attaaaaaag gtaattcaaa tgaaattgtt aaatgtaatt aattttgttt tcttgatgtt 4260
tgtttcatca tcttcttttg ctcaggtaat tgaaatgaat aattcgcctc tgcgcgattt 4320
tgtaacttgg tattcaaagc aatcaggcga atccgttatt gtttctcccg atgtaaaagg 4380
tactgttact gtatattcat ctgacgttaa acctgaaaat ctacgcaatt tctttatttc 4440
tgttttacgt gcaaataatt ttgatatggt aggttctaac ccttccataa ttcagaagta 4500
taatccaaac aatcaggatt atattgatga attgccatca tctgataatc aggaatatga 4560
tgataattcc gctccttctg gtggtttctt tgttccgcaa aatgataatg ttactcaaac 4620
ttttaaaatt aataacgttc gggcaaagga tttaatacga gttgtcgaat tgtttgtaaa 4680
gtctaatact tctaaatcct caaatgtatt atctattgac ggctctaatc tattagttgt 4740
tagtgctcct aaagatattt tagataacct tcctcaattc ctttcaactg ttgatttgcc 4800
aactgaccag atattgattg agggtttgat atttgaggtt cagcaaggtg atgctttaga 4860
tttttcattt gctgctggct ctcagcgtgg cactgttgca ggcggtgtta atactgaccg 4920
cctcacctct gttttatctt ctgctggtgg ttcgttcggt atttttaatg gcgatgtttt 4980
agggctatca gttcgcgcat taaagactaa tagccattca aaaatattgt ctgtgccacg 5040
tattcttacg ctttcaggtc agaagggttc tatctctgtt ggccagaatg tcccttttat 5100
tactggtcgt gtgactggtg aatctgccaa tgtaaataat ccatttcaga cgattgagcg 5160
tcaaaatgta ggtatttcca tgagcgtttt tcctgttgca atggctggcg gtaatattgt 5220
tctggatatt accagcaagg ccgatagttt gagttcttct actcaggcaa gtgatgttat 5280
tactaatcaa agaagtattg ctacaacggt taatttgcgt gatggacaga ctcttttact 5340
cggtggcctc actgattata aaaacacttc tcaggattct ggcgtaccgt tcctgtctaa 5400
aatcccttta atcggcctcc tgtttagctc ccgctctgat tctaacgagg aaagcacgtt 5460
atacgtgctc gtcaaagcaa ccatagtacg cgccctgtag cggcgcatta agcgcggcgg 5520
gtgtggtggt tacgcgcagc gtgaccgcta cacttgccag cgccctagcg cccgctcctt 5580
tcgctttctt cccttccttt ctcgccacgt tcgccggctt tccccgtcaa gctctaaatc 5640
gggggctccc tttagggttc cgatttagtg ctttacggca cctcgacccc aaaaaacttg 5700
atttgggtga tggttggcca tcgccctgat agacggtttt tcgccctttg acgttggagt 5760
ccacgttctt taatagtgga ctcttgttcc aaactggaac aacactcaac cctatctcgg 5820
gctattcttt tgatttataa gggattttgc cgatttcgga accaccatca aacaggattt 5880
tcgcctgctg gggcaaacca gcgtggaccg cttgctgcaa ctctctcagg gccaggcggt 5940
gaagggcaat cagctgttgc ccgtctcact ggtgaaaaga aaaaccaccc tggatccaag 6000
cttgcaggtg gcacttttcg gggaaatgtg cgcggaaccc ctatttgttt atttttctaa 6060
atacattcaa atatgtatcc gctcatgaga caataaccct gataaatgct tcaataatat 6120

tgaaaaagga agagtatgag tattcaacat ttccgtgtcg cccttattcc cttttttgcg 6180
gcattttgcc ttcctgtttt tgctcaccca gaaacgctgg tgaaagtaaa agatgctgaa 6240
gatcagttgg gcgcactagt gggttacatc gaactggatc tcaacagcgg taagatcctt 6300
gagagttttc gccccgaaga acgttttcca atgatgagca cttttaaagt tctgctatgt 6360
ggcgcggtat tatcccgtat tgacgccggg caagagcaac tcggtcgccg catacactat 6420
tctcagaatg acttggttga gtactcacca gtcacagaaa agcatcttac ggatggcatg 6480
acagtaagag aattatgcag tgctgccata accatgagtg ataacactgc ggccaactta 6540
cttctgacaa cgatcggagg accgaaggag ctaaccgctt ttttgcacaa catgggggat 6600
catgtaactc gccttgatcg ttgggaaccg gagctgaatg aagccatacc aaacgacgag 6660
cgtgacacca cgatgcctgt agcaatggca acaacgttgc gcaaactatt aactggcgaa 6720
ctacttactc tagcttcccg gcaacaatta atagactgga tggaggcgga taaagttgca 6780
ggaccacttc tgcgctcggc ccttccggct ggctggttta ttgctgataa atctggagcc 6840
ggtgagcgtg ggtctcgcgg tatcattgca gcactggggc cagatggtaa gccctcccgt 6900
atcgtagtta tctacacgac ggggagtcag gcaactatgg atgaacgaaa tagacagatc 6960
gctgagatag gtgcctcact gattaagcat tggtaactgt cagaccaagt ttactcatat 7020
atactttaga ttgatttaaa acttcatttt taatttaaaa ggatctaggt gaagatcctt 7080
tttgataatc tcatgaccaa aatcccttaa cgtgagtttt cgttccactg tacgtaagac 7140
ccccaagctt gtcgactgaa tggcgaatgg cgctttgcct ggtttccggc accagaagcg 7200
gtgccggaaa gctggctgga gtgcgatctt cctgacgctc gagcgcaacg caattaatgt 7260
gagttagctc actcattagg caccccaggc tttacacttt atgcttccgg ctcgtatgtt 7320
gtgtggaatt gtgagcggat aacaatttca cacaggaaac agctatgacc atgattacgc 7380
caagctttgg agcctttttt ttggagattt tcaacgtgaa aaaattatta ttcgcaattc 7440
ctttagttgt tcctttctat tccatggcgg ccgagatgca ttcattctgc gctttcaaag 7500
ctgatgacgg tccgtgtaaa gctatcatga aacgtttctt cttcaacatt ttcacgcgtc 7560
aatgtgaaga gttcatttac ggtggttgtg aaggtaacca gaaccggttc gaatctctag 7620
aggaatgtaa gaagatgtgc actcgtgatt ctgctagctc tgctagtggc gacttcgact 7680
acgagaaaat ggctaatgcc aacaaaggcg ccatgactga gaacgctgac gagaatgctt 7740
tgcaaagcga tgccaagggt aagttagaca gcgtcgcgac cgactatggc gccgccatcg 7800
acggctttat cggcgatgtc agtggtttgg ccaacggcaa cggagccacc ggagacttcg 7860
caggttcgaa ttctcagatg gcccaggttg gagatgggga caacagtccg cttatgaaca 7920
actttagaca gtaccttccg tctcttccgc agagtgtcga gtgccgtcca ttcgttttcg 7980
gtgccggcaa gccttacgag ttcagcatcg actgcgataa gatcaatctt ttccgcggcg 8040
ttttcgcttt cttgctatac gtcgctactt tcatgtacgt tttcagcact ttcgccaata 8100
ttttacgcaa caaagaaagc tagtgatctc ctaggaagcc cgcctaatga gcgggctttt 8160
tttttctggt atgcatcctg aggccgatac tgtcgtcgtc ccctcaaact ggcagatgca 8220
cggttacgat gcgcccatct acaccaacgt gacctatccc attacggtca atccgccgtt 8280
tgttcccacg gagaatccga cgggttgtta ctcgctcaca tttaatgttg atgaaagctg 8340
gctacaggaa ggccagacgc gaattatttt tgatggcgtt cctattggtt aaaaaatgag 8400
ctgatttaac aaaaatttaa tgcgaatttt aacaaaatat taacgtttac aatttaaata 8460
tttgcttata caatcttcct gtttttgggg cttttctgat tatcaaccgg ggtacatatg 8520
attgacatgc tagttttacg attaccgttc atcgattctc ttgtttgctc cagactctca 8580
ggcaatgacc tgatagcctt tgtagatctc tcaaaaatag ctaccctctc cggcattaat 8640
ttatcagcta gaacggttga atatcatatt gatggtgatt tgactgtctc cggcctttct 8700
cacccttttg aatctttacc tacacattac tcaggcattg catttaaaat atatgagggt 8760
tctaaaaatt tttatccttg cgttgaaata aaggcttctc ccgcaaaagt attacagggt 8820
cataatgttt ttggtacaac cgatttagct ttatgctctg aggctttatt gcttaatttt 8880
gctaattctt tgccttgcct gtatgattta ttggatgtt 8919

## Description

### CROSS-REFERENCE TO RELATED APPLICATIONS

This application claims the benefit of priority of U.S.S.N. 60/438,491, filed January 7, 2003, the contents of which are hereby incorporated by reference in its entirety.

### BACKGROUND

Phage display can be used to identify protein ligands that interact with a particular target. This technique uses bacteriophage particles as vehicles for linking candidate protein ligands to the nucleic acids encoding them. The coding nucleic acid is packaged within the bacteriophage, and generally the encoded protein on the phage surface. Phage display is described, for example, in Ladner et al., U.S. Patent No. 5,223,409; Smith (1985) Science 228:1315-1317; WO 92/18619; WO 91/17271; WO 92/20791; WO 92/15679; WO 93/01288; WO 92/01047; WO 92/09690; WO 90/02809; de Haard et al. (1999) J. Biol. Chem 274:18218-30; Hoogenboom et al. (1998) Immunotechnology 4:1-20; and Hoogenboom et al. (2000) Immunol Today 2:371-8. Other protein evaluation methods, e.g., protein arrays, can also be used to identify useful ligands.

A variety of scaffolds can be used as a template for identifying useful ligands. Useful scaffolds can include amino acid positions that contribute to a stable scaffold structure and other positions that can be varied to produce a binding site that interacts with a target.

### SUMMARY

Disclosed are libraries, vectors, phage particles, host cells, and methods for displaying a Kunitz domain. One exemplary library is a phage library that includes phage particles that include sufficient phage genes to produce a phage particles and a display protein that has a varied Kunitz domain. The Kunitz domain can vary in at least two interaction loops with respect to other members of the library. In one embodiment, the varied Kunitz domains can be displayed on filamentous phage at a low valency. Ion one embodiment, low valency is provided by a phage nucleic acid that includes a sequence encoding the display protein fused to a functional domain of a minor coat protein and a sequence that produces a counterpart protein that also includes the functional domain.

In one aspect, the invention features a library that includes a plurality of filamentous phage particles. Each phage particle of the plurality includes (i) a display protein that comprises a Kunitz domain, (ii) a nucleic acid that includes (a) optionally, phage genes sufficient to produce an infectious phage particle and (b) a sequence encoding the display protein. The Kunitz domain includes at least one varied amino acid position in each of the two interaction loops. The positions in the respective loops are varied among the particles of the plurality. The average number of copies of the Kunitz domain per phage particles of the plurality is less than 2.0.

The Kunitz domain can be at least 70, 75, 80, 85, 90, 95, 97, 98, 99, or 100% identical to a human Kunitz domain (e.g. LACI-K1) at amino acid positions that are not varied. In one embodiment, between one and fifteen, e.g., five and twelve amino acid positions are varied among the particles of the plurality. For example, at least amino acid positions corresponding amino acid positions 16, 17, 18, 19, 34, and 39 of human LACI-K1 are varied among the particles of the plurality. In another example, at least amino acid positions corresponding amino acid positions 11, 13, 15, 16, 17, 18, 19, 34, and 39 of human LACI-K1 are varied among the particles of the plurality. In still another example, at least six, seven or eight amino acid positions corresponding amino acid positions 11, 13, 15, 16, 17, 18, 19, 32, 34, 39, 40, and 46 of human LACI-K1 are varied among the particles of the plurality. Only amino acid positions corresponding amino acid positions 11, 13, 15, 16, 17, 18, 19, 34, 39, and 40 of human LACI-K1 may be varied among the particles of the plurality, or only 16, 17, 18, 19, 34, and 39, or only 11, 13, 15, 16, 17, 18, 19, 34, and 39, or other combinations.

Each Kunitz domain in the plurality may include at least 75, 80, 85, 90, 95, 97, 98, 99, or 100% of the Kunitz domains in the plurality can include Kunitz conserved or Kunitz highly conserved residues at some (e.g., 50, 60, 80, or 90%) or all varied positions. Each Kunitz domain in the plurality may include at least 75, 80, 85, 90, 95, 97, 98, 99, or 100% of the Kunitz domains in the plurality can include Kunitz conserved or Kunitz highly conserved residues at some (e.g., 50, 60, 80, or 90%) or all invariant positions. In one embodiment, amino acids at positions 32 and 46 are invariant.

The degree of variations can differ at different varied positions. For example, the amino acid position corresponding to amino acid position 40 can be varied between G and A. If position 40 is not varied, then it can be constrained to G or A, for example. Other varied positions can be varied, variously, among all amino acids, all non-cysteine amino acids, all amino acids except C and P, amino acids except C, P, and G, hydrophobics, aliphatics, hydrophilics, and charged.

In one embodiment, the display protein includes a functional domain of a minor coat protein fused to the Kunitz domain, and the phage genes include a gene that encodes the minor coat protein that is (i') not fused to a non-viral amino acid sequence of at least five amino acids or (ii') not fused to a varied amino acid sequence. For example, the phage genes can include a wild-type copy of the gene that encodes the endogenous minor coat protein of the bacteriophage. Generally, the phage genes can be wild-type copies or functional variants thereof.

In addition to the plurality of phage particles characterized above, other phage particles may be present, including, for example, inactive particles that lack a nucleic acid component.

In another aspect, the invention features a library that includes a plurality of phage particles. Each phage particle of the plurality includes (i) a display protein that comprises a Kunitz domain and at least a portion of the gene III phage coat protein, (ii) a functional gene III phage coat protein, and (iii) a nucleic acid that includes (a) optionally, phage genes sufficient to produce an infectious phage particle and (b) a sequence encoding the display protein. The Kunitz domain can include a sequence that is at least 50, 60, 70, 80, 85, 87, 90, 92, 94, 95, 96, 97, 98, 100% identical to
MHSFCAFKADX₁₁GX₁₃CX₁₅X₁₆X₁₇X₁₈X₁₉RFFFNIFTRQCEEFX₃₄YGGCX₃₉X₄₀ NQNRFESLEECKKMCTRDGA, at positions other than X (SEQ ID NO:10). For example,
X₁₁ is one of: A, D, E, F, G, H, I, K, L, M, N, P, Q, R, S, T, V, W, Y;
X₁₃ is one of: A, D, E, F, G, H, I, K, L, M, N, P, Q, R, S, T, V, W, Y;
X₁₅ is one of; A, D, E, F, G, H, I, K, L, M, N, Q, R, S, T, V, W, Y;
X₁₆ is one of: A, G, E, D, H, T;
X₁₇ is one of: A, D, E, F, G, H, I, K, L, M, N, Q, R, S, T, V, W, Y;
X₁₈ is one of: A, D, E, F, G, H, I, K, L, M, N, Q, R, S, T, V, W, Y;
X₁₉ is one of: A, D, E, F, G, H, I, K, L, M, N, P, Q, R, S, T, V, W, Y;
X₃₄ is one of: A, C, D, E, F, G, H, I, K, L, M, N, P, Q, R, S, T, V, W, Y;
X₃₉ is one of: A, C, D, E, F, G, H, I, K, L, M, N, P, Q, R, S, T, V, W, Y; and X₄₀ is one of: G, A.

At least two of X₁₁, X₁₃, X₁₅, X₁₆, X₁₇, X₁₈, X₁₉, X₃₄, X₃₉, and X₄₀ can vary among particles of the plurality. The at least two varied positions are typically in the first and second interaction loops of the Kunitz domain so that variation can be present in both loops. If an X position does not vary, it can, for example, be fixed to one of the amino acids listed above for a respective position. If an X position does vary, it can vary among the amino acid positions listed above, or among other possible combinations.

In one embodiment, the display protein includes the gene III coat protein stump. The phage genes include a gene that encodes a wild-type gene III coat protein.

In one embodiment, the library has a theoretical diversity of theoretical diversity of at least 10⁷, 10⁹, 10¹⁰, or 10¹¹ different Kunitz domains and/or fewer than 10¹⁵, 10¹⁴, 10¹³, 10¹², 10¹¹, or 10¹⁰ different Kunitz domains. In one embodiment, the theoretical diversity is between 10⁵-10¹¹ or between 10³ and 10⁵ different Kunitz domains.

In one embodiment, at least amino acid positions 15, 16, 17, 18, 34, and 39 are varied. In another embodiment, at least amino acid positions 11, 13, 15, 16, 17, 18, 19, 34, 39, and 40 are varied. In another embodiment, only those positions are varied, e.g., only 15, 16, 17, 18, 34, and 39, or 11, 13, 15, 16, 17, 18, 19, 34, 39, and 40, or 13, 15, 16, 17, 18, 19, 34, 39, and 40.

A library described herein can be used in a method of providing a nucleic acid encoding a Kunitz domain that interacts with a target. The method includes, for example, providing the library; contacting phage particles form the library to a target; and optionally, recovering nucleic acid from particles that interact with the target. For example, the target is immobilized, and the step of recovering includes separating particles that interact with the target from particles that do not interact with the target. Exemplary targets include proteases.

In another aspect, the invention features a nucleic acid that includes an open reading frame and a promoter operably linked to the open reading frame. The open reading frame encodes a display protein including: (i) a first element that contains a Kunitz domain; and (ii) a second element that contains a portion of a phage coat protein, wherein the portion is sufficient to physically associate the display protein with a phage particle.

The nucleic acid can be a vector that contains sufficient genetic information to produce infectious phage particles in the absence of helper phage. For example, the nucleic acid can include a set of phage genes sufficient to produce infectious phage particles. For example, the nucleic acid is a phage vector.

In one embodiment, the promoter is a regulatable promoter, e.g., an inducible promoter, e.g., the lac promoter.

In one embodiment, the coat protein is a minor coat protein, e.g., the gene III protein. The portion of the coat protein can include a portion that physically attaches to a phage particle, such as an anchor domain. In a preferred embodiment, the portion of the coat protein is the gene III anchor domain, or "stump." In another embodiment, the coat protein is a major coat protein, e.g., the gene VIII protein. In a preferred embodiment, the second element contains the full length, mature gene VIII coat protein. In one embodiment, the coat protein portion of (ii) is derived from one of: the gene IV protein, the gene VII protein, the gene IX protein.

In one embodiment, the Kunitz domain has a Kunitz conserved residue or a Kunitz highly conserved residue at at least 70, 75, 80, 85, 90, or 95% of amino acid positions (or all positions).

In one embodiment, the Kunitz domain has at least 30% identity with a Kunitz domain of a naturally occurring protein, e.g., to a protein that includes a Kunitz domain referred to herein. The Kunitz domain can have at least 40%, 50%, 60%, 70 %, 80%, 90%, 95%, or 98% identity to a Kunitz domain of a naturally occurring protein, e.g., a mammalian, e.g., primate, e.g., human protein, e.g., LAC-I.

For example, at least two cysteines are present in the Kunitz domain, and a disulfide can be formed between the cysteines. For example, if four cysteines are present, two disulfides form. Typically, six cysteines are present and three disulfides form between the cysteines.

In one aspect of the invention, the Kunitz domain comprises the following sequence: X₁-X₂-X₃-X₄-C₅-X₆-X₇-X₈-X₉-X₉ₐ-X₁₀-X₁₁-X₁₂-X₁₃-C₁₄-X₁₅-X₁₆-X₁₇-X₁₈-X₁₉-X₂₀-X₂₁-X₂₂-X₂₃-X₂₄-X₂₅-X₂₆-X₂₇-X₂₈-X₂₉-X₂₉ₐ-X_{29b}-X_{29c}-C₃₀-X₃₁-X₃₂-X₃₃-X₃₄-X₃₅-X₃₆-X₃₇-C₃₈-X₃₉-X₄₀-X₄₁-X₄₂-X₄₂ₐ-X_{42b}-X₄₃-X₄₄-X₄₅-X₄₆-X₄₇-X₄₈-X₄₉-X₅₀-C₅₁-X₅₂-X₅₃-X₅₄-C₅₅-X₅₆-X₅₇-X₅₈ (SEQ ID NO:2). At least four or six of C₅, C₁₄, C₃₀, C₃₈, C₅₁, and C₅₅, are independently cysteine. For example, all six are cysteine. If four are cysteine, the remainder of C₅, C₁₄, C₃₀, C₃₈, C₅₁, and C₅ can be an amino acid other than cysteine, or absent.

Each of X₁-X₄ is any amino acid, or absent. Each of X₆-X₁₃ is any amino acid but preferably not Cys. X₉ₐ is any amino acid but preferably not Cys, or absent. X₁₅-X_{29b} is any amino acid but preferably not Cys. Each of X₂₉ₐ, X_{29b}, X_{29c} is any amino acid, or absent. Each of X₃₁-X₃₇ is any amino acid but preferably not Cys. Each of X₃₉-X₅₀ is any amino acid but preferably not Cys. Each of X₅₂-X₅₄ is any amino acid but preferably not Cys. Each of X₅₆-X₅₈ is any amino acid, or absent.

In some embodiments, the number of amino acid residues between the cysteines of a Kunitz domain is increased or decreased by less than about 5 amino acids, e.g., by 5, 4, 3, 2, or 1 amino acids. For example, residues may be inserted or removed at or between X₆-X₁₃, X₁₅-X_{29c}, X₃₁-X₃₇, X₃₉-X₅₀, and X₅₂-X₅₄.

In one embodiment, in which either C₁₄ or C₃₀ is not cysteine, then both are not cysteine. In one embodiment, wherein C₁₄ and X₉ₐ are absent, X₁₂ can be G. In one embodiment, X₃₇ is G. In one embodiment, X₃₃ is F or Y. In one embodiment, X₄₅ is F or Y. For example, at least four cysteines are present and Cys-Cys bridges can be formed between two of C₅ and C₅₅, C₁₄ and C₃₈, and C₃₀ and C₅₁. Typically, six cysteines are present; and Cys-Cys bridges can be formed between C₅ and C₅₅, C₁₄ and C₃₈, and C₃₀ and C₅₁.

The set of phage genes can include a gene that encodes the phage coat protein of (ii), which is in addition to the copy that is linked to the Kunitz domain (encoded in the second element). For example, the set of genes can include a copy of the full length coat protein. In one embodiment, the nucleic acid encoding the portion of the coat protein in (ii) contains nucleotides that have been altered to prevent recombination with related sequences, e.g. a copy of the gene.

In one embodiment, each phage gene of the set is operably linked to the promoter endogenous to each gene.

In one embodiment, C₅, C₁₄, C₃₀, C₃₈, C₅₁, and C₅₅ are present, and X₉ₐ, X₂₉ₐ, X_{29b}, X_{29c}, X₄₂ₐ, X_{42b}, are absent.

In another embodiment, X₁₂ is G, X₃₃ is F, and X₃₇ is G

In one embodiment, the Kunitz domain can include one or more of the following properties: X₂₁ is one of F, Y, and W; X₂₂ is one of F and Y. X₂₃ is one of F and Y; X₃₅ is one ofY and W; X₃₆ is one of G and S. X₄₀ is one of G and A. X₄₃ is one of G and N. X₄₅ is one of F and Y.

In one embodiment, the Kunitz domain can include one or more of the following properties: X₁ is M; X₂ is H; X₃ is S; X₄ is F; X₆ is A; X₇ is F; X₈ is K; X₉ is A; X₁₀ is D; X₂₀ is R; X₂₁, X₂₂, X₂₃ are each F; X₂₄ is N; X₂₅ is I; X₂₆ is F; X₂₇ is T; X₂₈ is R; X₂₉ is Q; X₃₁ is E; X₃₅ is Y; X₃₆ is G; X₄₁ is N; X₄₂ is Q; X₄₃ is N; X₄₄ is R; X₄₅ is F; X₄₇ is S; X₄₈ is L; X₄₉ and X₅₀ are each E; X₅₂ and X₅₃ are each K; X₅₄ is M; X₅₆ is T; X₅₇ is R; and X₅₈ is D.

In one embodiment, X₁₅, X₁₇, X₁₈, X₄₀, X₄₆ are each any amino acid except proline; and X₁₆ is one of A, G, E, D, H, T.

In one embodiment, X₃₂ is E; X₃₄ is I; X₃₉ is E; X₄₀ is G; and X₄₆ is E.

In one embodiment, the nucleic acid further includes a selectable marker (e.g., antibiotic resistance gene, amp gene).

In another aspect, the invention features a library comprising a plurality of nucleic acids, wherein each nucleic acid of the plurality can include one or more of the features described herein. In one embodiment, the Kunitz domain sequence varies among nucleic acids of the plurality.

The plurality can contain nucleic acids that encode at least 10⁷,10⁹, 10¹⁰, or 10¹¹ different Kunitz domains and/or fewer than 10¹⁵, 10¹⁴, 10¹³, 10¹²10¹¹, or 10¹⁰ different Kunitz domains. In one embodiment, the plurality contains nucleic acids that encode at least 10⁵-10¹¹ different Kunitz domains. The plurality can be characterized by a theoretical diversity of at least 10⁷, 10⁹, 10¹⁰, or 10¹¹ different Kunitz domains and/or fewer than 10¹⁵ 10¹⁴, 10¹³, 10¹², 10¹¹, or 10¹⁰ different Kunitz domains. In one embodiment, the theoretical diversity is between 10⁵-10¹¹ different Kunitz domains.

In one embodiment, the invention features a library that contains a plurality of nucleic acids, wherein C₅, C₁₄, C₃₀, C₃₈, C₅₁, and C₅₅ are present, and X₉ₐ, X₂₉ₐ, X_{29b}, X_{29c}, X₄₂ₐ, X_{42b}, are absent in each nucleic acid, and wherein the Kunitz domain sequence varies at least at two of the positions corresponding to X₃₂, X₃₄, X₃₉, X₄₀ and X₄₆ of (SEQ ID NO:2) among members of the plurality.

In one embodiment, the Kunitz domain sequence is invariant at one or more positions corresponding to X₁₁, X₁₂, X₁₅, X₁₆, X₁₇, X₁₈, and X₁₉ (of SEQ ID NO: 2). In another embodiment, the Kunitz domain sequence varies at one or more positions corresponding to X₁₁, X₁₂, X₁₅, X₁₆, X₁₇, X₁₈, and X₁₉ of SEQ ID NO:2 among members of the plurality. In one embodiment, the Kunitz domain sequence is invariant at one or more positions corresponding to X₃₂, X₃₄, X₃₉, X₄₀ and X₄₆ of SEQ ID NO:2.

In one embodiment, the display protein includes a plurality of Kunitz domains, e.g., a plurality of varied Kunitz domains, or a varied Kunitz domain and at least another varied sequence.

In another aspect, the invention features a host cell comprising a nucleic acid, wherein the nucleic acid contains one or more of the features described herein. The host cell can be a bacterial cell, e.g., an E. *coli* cell.

In another aspect, the invention features a library comprising a plurality of host cells, wherein each host cell of the plurality comprises a nucleic acid that has one or more of the features described herein. In one embodiment, the Kunitz domain sequence varies among nucleic acids of the plurality.

In one aspect, the invention features a phage particle that contains a nucleic acid, wherein the nucleic acid can include one or more of the features described herein. In one embodiment, the particle contains the Kunitz domain physically attached to the surface.

In another aspect, the invention features a library containing a plurality of phage particles, wherein each phage particle of the plurality comprises a nucleic acid that can include one or more of the features described herein. In one embodiment, the Kunitz domain sequence differs among nucleic acids of the plurality.

In one embodiment, the plurality of phage particles contains at least 10³ particles that each include a nucleic acid encoding a different display protein. Preferably, the plurality of phage particles comprises at least 10⁶ particles that each include a nucleic acid encoding a different display protein. More preferably, the plurality of phage particles comprises at least 10⁹ particles that each include a nucleic acid encoding a different display protein. This actual measure of diversity can be less than the theoretical diversity of the library.

In one embodiment, at least 20%, 40%, 60%, or 80% of the phage particles of the plurality of phage particles includes the display protein of the respective phage particle physically attached to the phage particle. In one embodiment, the average copy number of the display protein is less than 3, e.g., less than 2.5, 2.0, 1.7, 1.5, or 1.2. If, however, particles that do not include a display protein but include an appropriate nucleic acid component are included in the plurality, the average copy number of the display protein can be less than 2, 1.5, 1.2, 1.1., 1.0, or 0.9. For example, the average copy number is between 2.4 and 0.5, or 1.8 and 0.5, or 1.4 and 0.5.

In some cases, it may be useful to evaluate a plurality of phage particles which excludes particles that do not include a display protein. Accordingly, the average copy number could not be less than one. In such cases, the average copy number of the display protein is less than 3, e.g., less than 2.5, 2.0, 1.7, 1.5, or 1.2, e.g., between 2.5 and 1.0 or 1.5 and 1.0.

A library of phage particles can be prepared in a liquid composition, e.g., an aqueous composition. The composition can provide an oxidizing environment, e.g., favoring disulfide formation within Kunitz domains. The composition can be non-viscous or has a low enough viscosity to enable liquid manipulation (e.g., pipetting 10, 5, or fewer microliters). When Kunitz domains are selected at random, the library can include at least 30, 40, 50, 70, 75, 80, 85, 90, or 95% domains that are folded. One method for determine the fraction of folded domains is to express the Kunitz domains in a cell with an epitope tag, and to perform Western blots on the soluble fraction of crude lysates of the cells. Detectable levels of the epitope tag (at the appropriate molecular weight) in the soluble fractions indicates that a folded Kunitz domain was produced. See, e.g., Davidson et al. (1994) ProcNatl Acad Sci USA. 91(6):2146-50.

In another aspect, the invention features a method that includes: (i) providing the library that includes one or more of the features described herein, (ii) selecting a set of phage particles that bind to a target using the display protein. The method can be used to select phage that encode a target binding protein from a plurality of phage particles.

In various embodiments, at least 10%, 20%, or 40%, more preferably, at least 60%, most preferably, at least 80% of the phage particles display the display protein on the surface.

The selecting can include: (a) forming a mixture containing the phage particles, a target, and a support, and (b) separating phage that do not bind to the target from the phage-immobilized target complexes.

In one embodiment, the target is a protease, e.g., an active protease or an inactive protease. Inactive proteases include proteases with amino acid alterations (e.g., substitutions, insertions, or deletions) that partially or completely reduce protease activity.

In one aspect, the invention features a method that includes: (a) providing a plurality of nucleic acids, wherein the plurality includes one or more of the features described herein; (b) introducing at least some nucleic acids of the plurality into host cells; and (c) assembling of phage particles that package the introduced nucleic acids under conditions, wherein at least some particles incorporate the display protein encoded by a respective introduced nucleic acid. The method can be used to provide a phage library.

In embodiments in which the nucleic acid contains a regulatable promoter, the method can further include propagating the library under conditions in which the regulatable promoter is repressed.

In one embodiment, helper phage are not introduced into the host cells.

In another aspect, the invention features a method that includes: (i) providing a phage library, wherein the library can include one or more of the features described herein; (ii) selecting a phage particle that displays a display protein that binds to the target; and (iii) recovering the nucleic acid of the selected phage particle, thereby identifying a display protein that binds a target. In one embodiment, the method further includes expressing a binding polypeptide that includes the Kunitz domain of the identified display protein. The method can further include purifying the binding polypeptide, formulating the binding polypeptide as a pharmaceutical composition, and administering the binding polypeptide (e.g., as such a pharmaceutical composition) to a subject, e.g., a mammal, e.g., a human. The method can be used to identifying a display protein that binds a target from a plurality of display proteins. Information about an identified protein can be transmitted, e.g., in digital form, or received. The recipient can produce a protein based on the information.

In another aspect, the invention features a nucleic acid that includes: (a) a set of phage genes sufficient to produce an infectious phage particle, (b) an open reading frame and (c) a promoter operably linked to the open reading frame. The open reading frame encodes a display protein that includes: (i) a first element that contains a Kunitz domain; and (ii) a second element that includes one or more amino acids that can physically associate the display protein with a phage particle. The second element can be, e.g., a cysteine that can form a disulfide with a cysteine on the phage particle, a sequence of amino acid that non-covalently interacts with the phage particle (e.g., for fos-jun interaction), or all or a part of a phage coat protein. The nucleic acid can be used as described herein.

The invention also features a Kunitz domain-containing protein, e.g., a protein that includes a Kunitz domain identified by a method described herein. For example, the protein can be less than 200, 100, or 70 amino acids in length. It can include single Kunitz domain or multiple Kunitz domains. The Kunitz domain of the protein can include:
MHSFCAFKADX₁₁GX₁₃CX₁₅X₁₆X₁₇X₁₈X₁₉RFFFNIFTRQCEEFX₃₄YGGCX₃₉X₄₀NQNRF ESLEECKKMCTRDGA, at positions other than X (SEQ ID NO:10), but differ from the sequence of LACI-K1 by at least one amino acid residue, e.g., at least five, six, seven, or eight of positions 11, 13, 15, 16, 17, 18, 19, 34, and 39. For example,
X₁₁ is one of: A, D, E, F, G, H, I, K, L, M, N, P, Q, R, S, T, V, W, Y;
X₁₃ is one of: A, D, E, F, G, H, I, K, L, M, N, P, Q, R, S, T, V, W, Y;
X₁₅ is one of: A, D, E, F, G, H, I, K, L, M, N, Q, R, S, T, V, W, Y;
X₁₆ is one of: A, G, E, D, H, T;
X₁₇ is one of: A, D, E, F, G, H, I, K, L, M, N, Q, R, S, T, V, W, Y;
X₁₈ is one of: A, D, E, F, G, H, I, K, L, M, N, Q, R, S, T, V, W, Y;
X₁₉ is one of: A, D, E, F, G, H, I, K, L, M, N, P, Q, R, S, T, V, W, Y;
X₃₄ is one of: A, C, D, E, F, G, H, I, K, L, M, N, P, Q, R, S, T, V, W, Y;
X₃₉ is one of: A, C, D, E, F, G, H, I, K, L, M, N, P, Q, R, S, T, V, W, Y; and X₄₀ is one of: G, A.

The term "polypeptide" refers to a polymer of three or more amino acids linked by a peptide bond. The polypeptide may include one or more unnatural amino acids. Typically, the polypeptide includes only natural amino acids. The term "peptide" refers to a polypeptide that is between three and thirty-two amino acids in length.

A "protein" can include one or more polypeptide chains. Accordingly, the term "protein" encompasses polypeptides and peptides. A protein or polypeptide can also include one or more modifications, e.g., a glycosylation, amidation, phosphorylation, and so forth.

The term "display protein" refers a protein, other than an unmodified viral coat protein, physically associated with the nucleic acid that encodes it. In the case of phage display, the display protein is physically associated with the phage particle that packages the nucleic acid that encodes it, and also includes an amino acid sequence of at least three amino acids that is heterologous to the bacteriophage. The heterologous region, more typically, includes a scaffold domain, e.g., a Kunitz domain. The physical association can be mediated, e.g., by one or more covalent bonds, e.g., one or more peptide bonds or a disulfide bond. In one embodiment, a display protein includes at least a functional domain of a phage coat protein, such that the display protein is incorporated into the phage particle

The term "viral particle" encompasses viruses that include a sufficient genetic information to produce progeny particles in a host cell and viral particles that can enter cells, but cannot produce progeny. The term "bacteriophage particle" encompasses bacteriophage that include a sufficient genetic information to produce progeny particles in a host cell and phage particles that can enter cells, but cannot produce progeny. Hence, the term "bacteriophage particle" includes both particles that package a phagemid and particles that package a complete phage genome.

A "human Kunitz domain library" refers to a library that includes different Kunitz domains or nucleic acid encoding such domains, wherein the invariant amino acid positions in the library are at least 85% identical to a particular human Kunitz domain. Human Kunitz domain libraries can be at least 85, 87, 90, 92, 93, 94, 95, 96, 97, 98, or 100% identical to a particular human Kunitz domain at the invariant amino acid positions.

A "LACI-K1 domain library" refers to a library that includes different Kunitz domains or nucleic acid encoding such domains, wherein the invariant amino acid positions in the library are at least 85% identical to human LACI-K1. LACI-K1 domain libraries can be at least 85, 87, 90, 92, 93, 94, 95, 96, 97, 98, or 100% identical to LACI-K1 at the invariant amino acid positions. For example, a 100% LACI-K1 domain library refers to a library in which the invariant amino acid positions in the library 100% identical to human LACI-K1. The same nomenclature can be used to refer to libraries that have a corresponding relationship to other Kunitz domains, e.g., other human Kunitz domains, e.g., a domain described herein.

An "expression system" is a configuration of nucleic acid sequences that includes an open reading frame and a promoter such that the open reading frame is operably linked to the promoter and the open reading frame can be expressed as a transcript that can be translated.

Calculations of homology or sequence identity between sequences (the terms are used interchangeably herein) are performed as follows. The "percent identity" between the two sequences is a function of the number of identical positions shared by the sequences, taking into account the number of gaps, and the length of each gap, which need to be introduced for optimal alignment of the two sequences. The comparison of sequences and determination of percent identity between two sequences can be accomplished using a mathematical algorithm. The percent identity between two amino acid or nucleotide sequences can be determined using the algorithm of Needleman and Wunsch ((1970) J. Mol. Biol. 48:444-453) algorithm which has been incorporated into the GAP program in the GCG software package, using a Blossum 62 matrix and a gap weight of 12, a gap extend penalty of 4, and a frameshift gap penalty of 5.

Generally, to determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, 60%, and even more preferably at least 70%, 80%, 90%, 100% of the length of the reference sequence (e.g., at least 51, 55, 57 or 58 amino acids). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology").

The GAP program is used to identify the optimal alignment between a query sequence and a reference Kunitz domain sequence (e.g., the LACI-K1 sequence) to identify "corresponding" amino acid positions.

The "interaction loops" of a Kunitz domain refer to the first interaction loop which includes amino acid positions corresponding to amino acid positions 11 to 19 of LACI-K1, and the second interaction loop which includes amino acid positions corresponding to amino acid positions 32 to 40 of LACI-K1.

The details of one or more embodiments of the invention are set forth in the accompanying drawings and the description below. Other features, objects, and advantages of the invention will be apparent from the description and drawings, and from the claims. All cited patents, patent applications, and references (including references to public sequence database entries) are incorporated by reference in their entireties for all purposes. USSN 60/438,491, US-2003-0129659-A1,and 10/656,350 are incorporated by reference in their entireties for all purposes.

### DESCRIPTION OF DRAWINGS

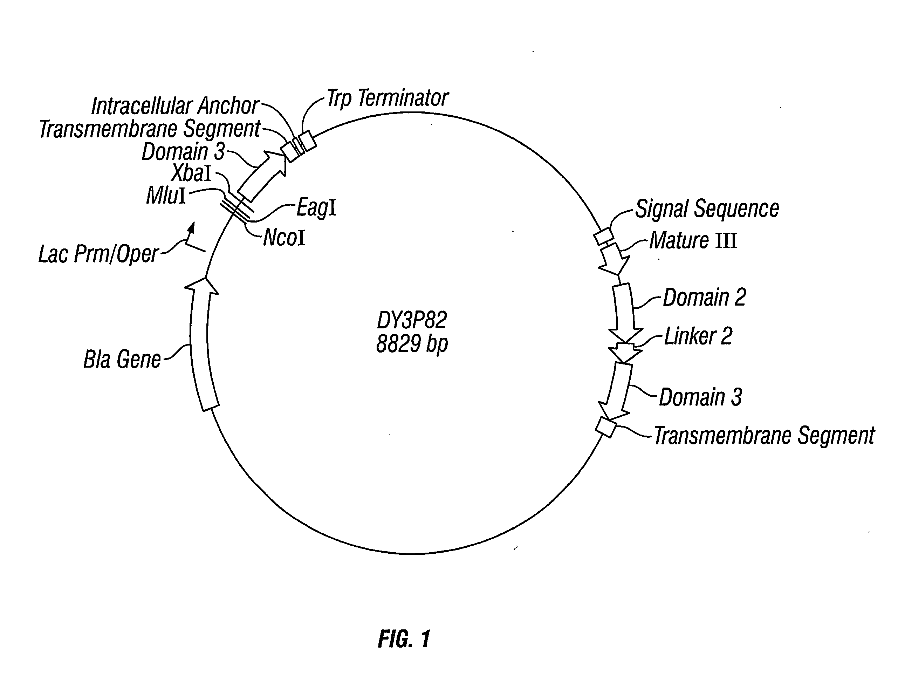

**FIG 1** is a diagram of an exemplary phage vector, DY3P82, which can be used for an exemplary LACI-K1-derived Kunitz domain library.

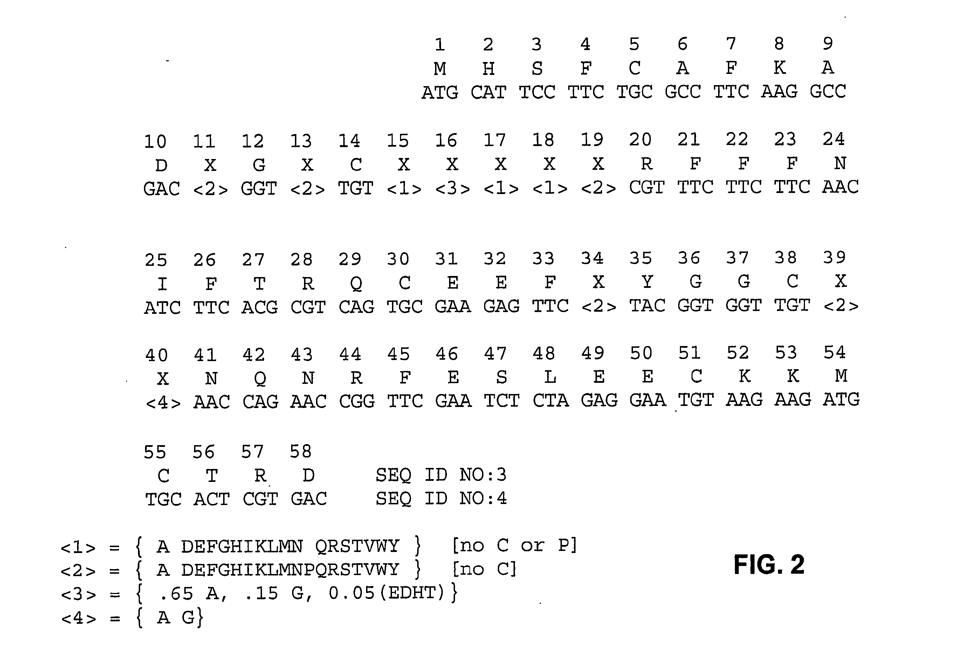

**FIG 2** is a diagram of the design of a first exemplary library. The amino acid sequence is listed as SEQ ID NO:4. An approximate rendition of the nucleic acid sequence is listed as SEQ ID NO:5. (However, the use of trinucleotide-varied codons may not be embodied in the nucleic acid sequence listing).

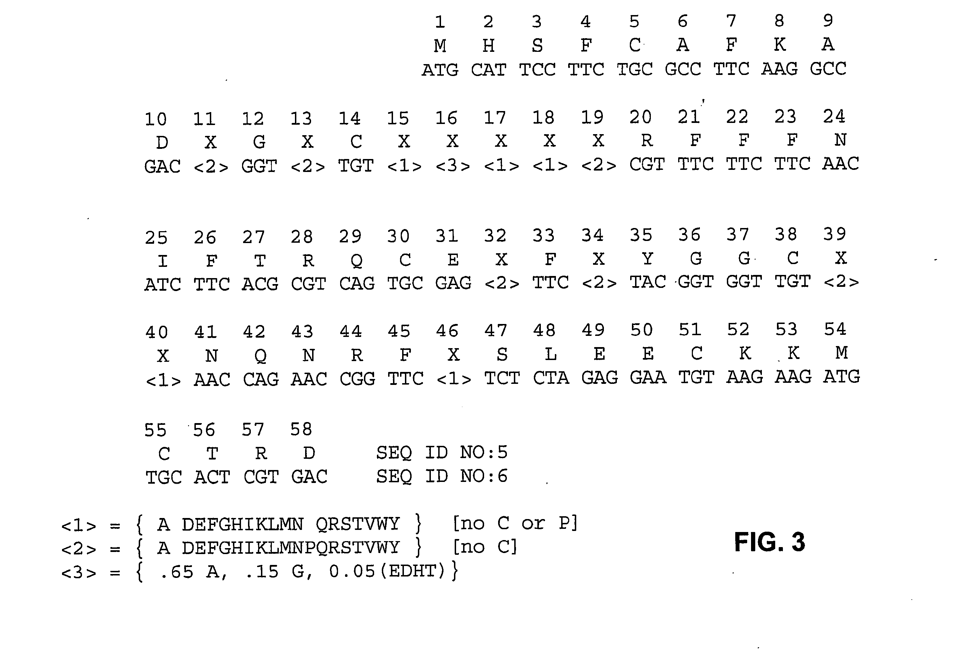

**FIG 3** is a diagram of the design of a second exemplary library. The amino acid sequence is listed as SEQ ID NO:6. An approximate rendition of the nucleic acid sequence is listed as SEQ ID NO:7. (Again, the use of trinucleotide-varied codons may not be embodied in the nucleic acid sequence listing).
   For FIGs. 2 and 3, amino acid position numbers are listed above each amino acid. Corresponding nucleotides, restriction enzyme sites, and sites of variation are listed below each amino acid. Variable positions also contain a second number, indicating the possible number of amino acids allowed at that position.

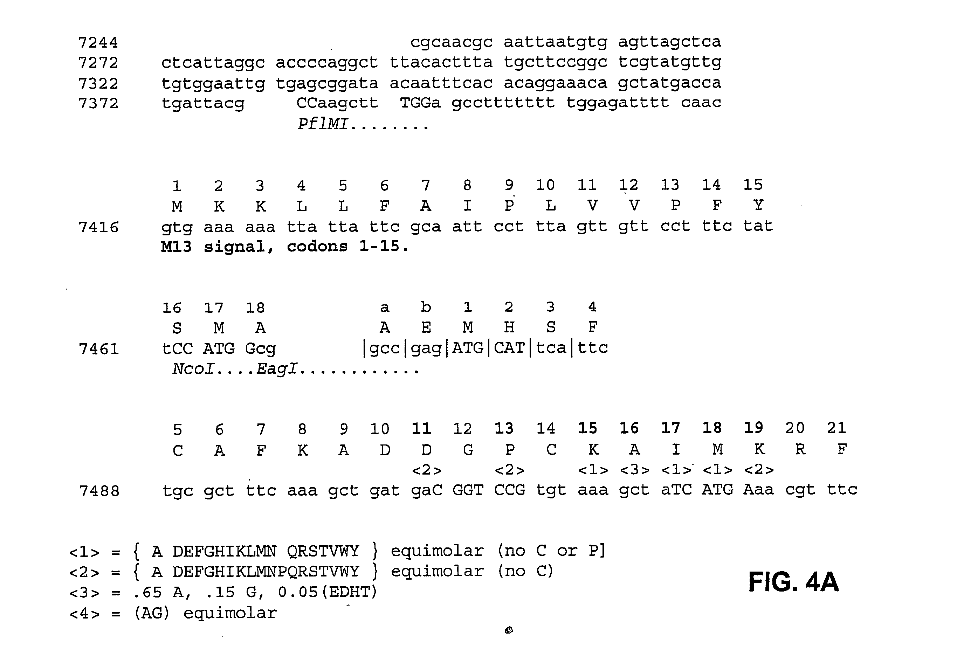

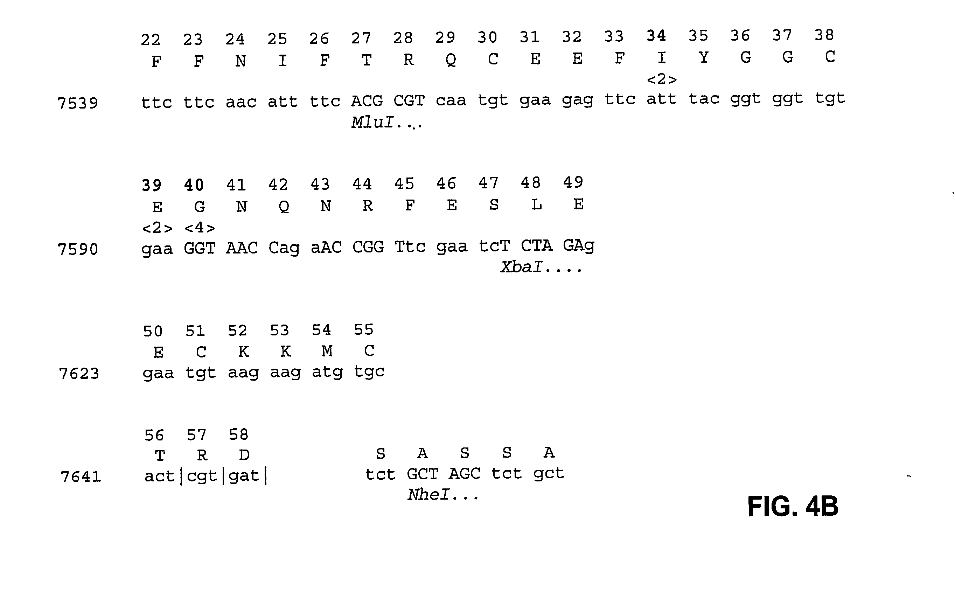

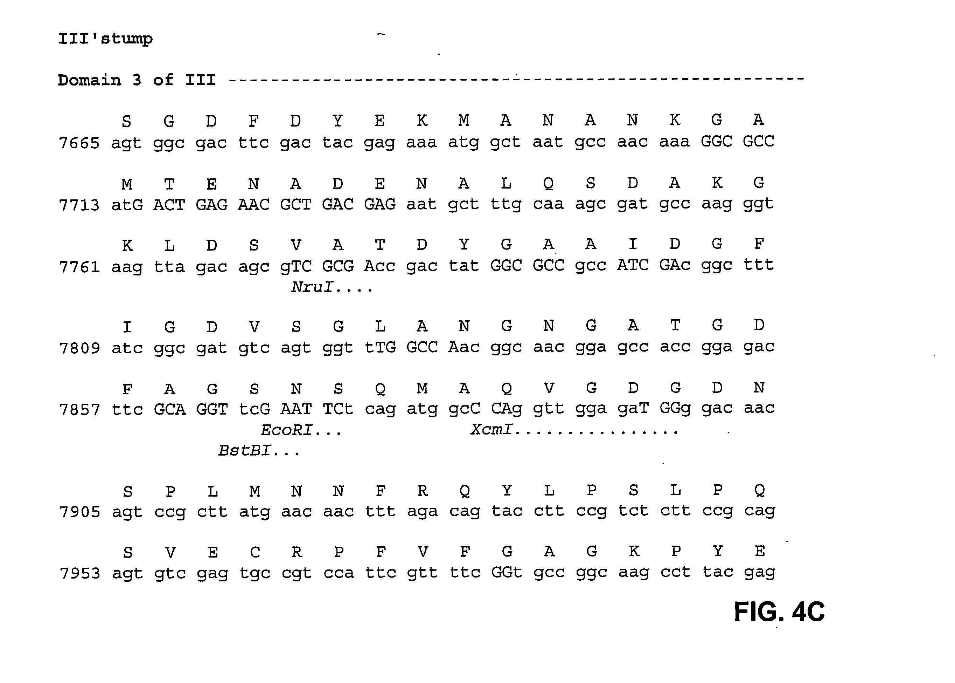

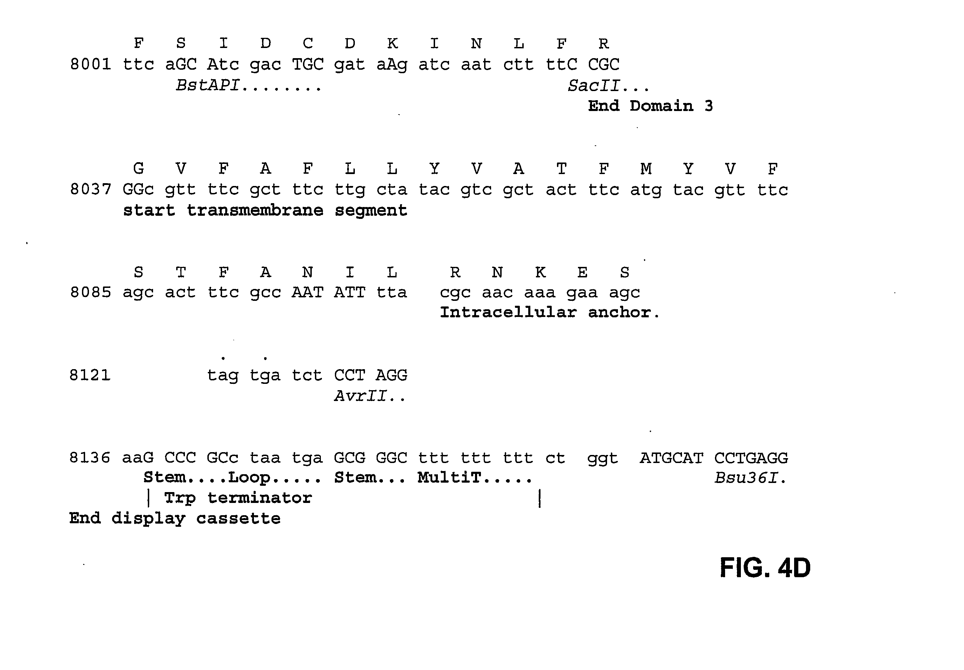

**FIG. 4A****, B, C,** and **D** illustrates the DNA sequence (SEQ ID NO:8) of the display cassette of the exemplary phage vector DY3P82_LACIK1, and the amino acid sequence (SEQ ID NO:9) of the varied display protein. (The use of trinucleotide-varied codons may not be embodied in the nucleic acid sequence listing). Bases 7244 through 7415 contain the PlacZ promoter and a ribosome binding site. Bases 7416 - 7469 encode the 18 amino acid signal sequence of M13 iii. Signal peptidase cleaves after Ala18. The bases 7470-7475 encode the amino acids Ala-Glu, here labeled "a" and "b". These allow efficient cleavage by signal peptidase I. Bases 7476-7649 encode the LACI K1 domain, shown here with the wild-type sequence and numbered 1 through 58. The variegated positions (11, 13, 15, 16, 17, 18, 19, 34, 39, and 40) are shown. The restriction sites shown are unique within DY3P82_LACIK1.

### DETAILED DESCRIPTION

In one aspect, the invention provides a phage particle that displays a Kunitz domain. The particle can be a member of a library of phage particles in which the Kunitz domains of respective members are varied with respect to one another. Phage display libraries can be used to identify a Kunitz domain that can bind to a particular target, e.g., a target protease. The Kunitz domain may also inhibit the activity of the target protease.

Phage display libraries are collections of phage particles that (i) include a varied display protein on the particle surface and (ii) contain the nucleic acid encoding the display protein. The physical association between the display protein and its encoding nucleic acid is convenient for the rapid isolation of target-binding proteins.

The phage particles can present the display protein at a desired valency. For example, the particles can present the display protein at a limited valency, e.g., in a copy number that is less than the maximal possible copy number. For example, if the display protein is attached to the phage particle by at least a portion of the gene III protein, the maximal possible copy number is about five. Accordingly, the valency of the displayed Kunitz domain on the phage particle can limited, e.g., to less than five, four, three, or two copies per particle.

In a library, the average or median valency can be, e.g., less than or equal to 4, 3, 2, 1.5, 1.4, 1.25, 1.1, or 1.0, or between 0.25-2, 0.3 and 1.8, 0.3 and 1.5, or 0.5 and 1.5. Reduced valency favors the selection of high specificity binders. Monovalent display (e.g., an average valency less than 1.4) can be used.

One method of achieving limited valency is use a display protein that includes at least a functional portion of a minor coat protein. Another copy of the minor coat protein (or a functional portion) thereof -- a counterpart protein -- is also expressed while phage particles are being produced so that, on average, fewer display proteins are incorporated into a given particle that the maximum possible copy number of the minor coat protein. For example, if the gene III coat protein of filamentous phage is used, the typical maximum possible copy number is about five. Expression of display proteins as fusions to at least the anchor domain of the gene III coat protein concurrent with expression of the wild-type gene III coat protein can result in phage particles with less than five, four, three, or two display proteins per particle.

The nucleic acid sequence that encodes the display can be operably linked to a promoter that gives a desired level of expression relative to other phage components. For example, a regulatable promoter can be used, e.g., to allow control of the ratio of expression of the display protein to its counterpart.

Without intending to be bound by theory, the display protein and its counterpart may compete for incorporation into the phage particle. One consequence of controlling the ratio of expression of the display protein and its counterpart is control of the valency of the display protein.

The sequences that encode the corresponding functional domains of the display protein and the counterpart protein can differ. For example, if the corresponding functional domains have identical amino acid sequences, codon choice can be varied to produce different coding sequences. For example, one of the two can include synthetic codons selected to prevent recombination between the nucleic acid sequence encoding the display protein and the nucleic acid sequence encoding the counterpart protein, which may use natural codons. The scenario can also be reversed, e.g., the nucleic acid encoding the display protein can use synthetic codons to encode the coat protein or fragment thereof. For example, the two sequences can differ at between 5 and 95, 5 to 60, or 20 and 50% cordons.

### Exemplary Kunitz Domains

As used herein, a "Kunitz domain" is a polypeptide domain that includes at least 51 amino acids and contains at least two, and more typically three, disulfide bonds. The domain is folded such that, if present, the first and sixth cysteines, the second and fourth, and the third and fifth cysteines form disulfide bonds. For example, in an exemplary Kunitz domain having 58 amino acids, disulfides are form between the cysteines at position 5 and 55, 14 and 38, and 30 and 51.

In implementations in which two disulfides are present, disulfide bonds can be formed between a corresponding subset of cysteines. The spacing between respective cysteines can be within 7, 5, 4, 3 or 2 amino acids of the following spacing: 5 to 55, 14 to 38, and 30 to 51.

In SEQ ID NO:2, disulfides bonds link at least two of: 5 to 55, 14 to 38, and 30 to 51, as follows: X₁-X₂-x₃-X₄-G₅-X₆-X₇-X₈-X₉-X₉ₐ-X₁₀-X₁₁-X₁₂-X₁₃-C₁₄-X₁₅-X₁₆-X₁₇-X₁₈-X₁₉-X₂₀-X₂₁-X₂₂-X₂₃-X₂₄-X₂₅-X₂₆-X₂₇-X₂₈-X₂₉-X₂₉ₐ-X_{29b}-X₂₉C₃₀-X₃₁-X₃₂-X₃₃-X₃₄-X₃₅-X₃₆-X₃₇-C₃₈-X₃₉-X₄₀-X₄₁-X₄₂-X₄₂ₐ-X_{42b}-X₄₃-X₄₄-X₄₅-X₄₆-X₄₇-X₄₈-X₄₉-X₅₀-C₅₁-X₅₂-X₅₃-X₅₄-C₅₅-X₅₆-X₅₇-X₅₈ (SEQ ID NO:2) .

The number of disulfides may be reduced by one, but, generally, none of the standard cysteines shall be left unpaired. Thus, if one cysteine is changed, then a compensating cysteine is added in a suitable location or the matching cysteine is also replaced by a non-cysteine. For example, *Drosophila* funebris male accessory gland protease inhibitor has no cysteine at position 5, but has a cysteine at position -1 just before position 1; presumably this forms a disulfide to Cys₅₅.

In some embodiments, C₁₄ and C₃₀ can be changed to amino acids other than cysteine, but preferably if one of these residues is changed, both are changed. If C₁₄ is present and X₉ₐ is absent, then X₁₂ can be Gly. In some embodiments, X₃₇ is Gly. In some embodiments, X₃₃ is Phe or Tyr, and X₄₅ is Phe or Tyr. In some embodiments, Cys₁₄ and Cys₃₈ are replaced, and the requirement of Gly₁₂, (Gly or Ser)₃₇, and Gly₃₆ is dropped.

From zero to many residues can be located to either end of a Kunitz domain. These residues can constitute, e.g., one or more domains (e.g., other Kunitz and non Kunitz display domains) or other amino acid sequences.

Natural Kunitz domains are generally highly stable domains. Kunitz domains can bind in the active sites of their respective protease targets so that a peptide bond (the "scissile bond") of the Kunitz domain is: 1) not cleaved, 2) cleaved very slowly, or 3) cleaved to no effect because the structure of the inhibitor prevents release or separation of the cleaved segments. Disulfide bonds generally act to hold the protein together even if exposed peptide bonds are cleaved.

From the residue on the amino side of the scissile bond, and moving away from the bond, residues are conventionally called P1, P2, P3, etc. Residues that follow the scissile bond are called P1', P2', P3', etc. It is generally accepted that each serine protease has sites (comprising several residues) S1, S2, etc., that receive the side groups and main chain atoms of P1, P2, etc. of the substrate or inhibitor and sites S1', S2', etc. that receive the side groups and main chain atoms of P1', P2', etc. of the substrate or inhibitor. It is the interactions between the S sites and the P side groups and main chain atoms that give the protease specificity with respect to substrates and the inhibitors specificity with respect to proteases. Because the fragment having the new amino terminus leaves the protease first, many designing small molecule protease inhibitors have concentrated on compounds that bind sites S1, S2, S3, etc.

Typically, X₁₅ or the amino acid position corresponding to position 15 of LACI-K1 is equivalent to P1 (described above), X₁₆ or the amino acid position corresponding to position 16 of LACI-K1 is equivalent to P1', X₁₇ or the amino acid position corresponding to position 17 of LACI-K1 is equivalent to P2', X₁₈ or the amino acid position corresponding to position 18 of LACI-K1 is equivalent to P3', and X₁₉ or the amino acid position corresponding to position 19 of LACI-K1 is equivalent to P4'. As discussed below, one or more of S1, S2, S3, P1', P2', P3', and P4' can be varied.

Exemplary human Kunitz domains include the three Kunitz domains of LACI (Wun et al., (1988) J. Biol. Chem. 263(13):6001-6004; Girard et al., (1989) Nature, 338:518-20; Novotny et al, (1989) J. Biol. Chem., 264(31):18832-18837); the two Kunitz domains of Inter-α-Trypsin Inhibitor, APPI (Alzheimer's amyloid β-protein precursor inhibitor) (Kido et al., (1988) J. Biol. Chem., 263(34):18104-18107), a Kunitz domain from collagen, and the three Kunitz domains of TFPI-2 (Sprecher et al., (1994) Proc. Nat. Acad. USA, 91:3353-3357).

LACI is a human serum phosphoglycoprotein that contains three Kunitz domains:

MIYTMKKVHA LWASVCLLLN LAPAPLNADS EEDEEHTIIT DTELPPLKLM
HSFCAFKADD GPCKAIMKRF FFNIFTRQCE EFIYGGCEGN QNRFESLEEC
KKMCTRDNAN RIIKTTLQQE KPDFCFLEED PGICRGYITR YFYNNQTKQC
ERFKYGGCLG NMNNFETLEE CKNICEDGPN GFQVDNYGTQ LNAVNNSLTP
QSTKVPSLFE FHGPSWCLTP ADRGLCRANE NRFYYNSVIG KCRPFKYSGC
GGNENNFTSK QECLRACKKG FIQRISKGGL IKTKRKRKKQ RVKIAYEEIF
VKNM (SEQ ID NO:3)

The signal sequence is located at amino acids 1-28. The three Kunitz domains within LACI are referred to as LACI-K1 (residues 50 to 107 of SEQ ID NO:3), LACI-K2 (residues 121 to 178 of SEQ ID NO:3), and LACI-K3 (213 to 270 of SEQ ID NO:3). The cDNA sequence of LACI is reported in Wun et al. (J. Biol. Chem., 1988, 263(13):6001-6004). Girard et al. (Nature, 1989, 338:518-20) reports mutational studies in which the P1 residues of each of the three Kunitz domains were altered. LACI-K1 inhibits Factor VIIa when Factor VIIa is complexed to tissue factor. LACI-K2 inhibits Factor Xa.

Herein, the residues of exemplary Kunitz domains are numbered by reference to the LACI-K1 (Kunitz domain 1 of LACI):

MHSFCAFKAD DGPCKAIMKR FFFNIFTRQC EEFIYGGCEG
NQNRFESLEE CKKMCTRD (SEQ ID NO:1)

The first cysteine residue of the LACI-K1 Kunitz domain is residue 5 and the last cysteine is residue 55. The amino acid positions in a Kunitz domain can also be referenced with respect to their correspondence with amino acids in LACI-K1, using the optimal alignment provided by the GAP program (see above).

Kunitz domains of this invention can be at least 30, 40, 50, 60, 70, 80, or 90% identical to LACI-K1. Other Kunitz domains of this invention are homologous (e.g., at least 30, 40, 50, 60, 70, 80, or 90% identical) to other naturally-occurring Kunitz domains (e.g., a Kunitz domain described herein), particularly to other human Kunitz domains.

The three dimensional molecular structures of many Kunitz domains are known at high resolution. See, e.g., Eigenbrot et al. (1990) Protein Engineering 3:591-598 and Hynes et al. (1990) Biochemistry 29:10018-10022. One exemplary X-ray structural model of the BPTI Kunitz domain is deposited in the Brookhaven Protein Data Bank as "6PTI".

More than seventy Kunitz domain sequences are known. Proteins containing exemplary Kunitz domains include the following, with SWISS-PROT Accession Numbers in parentheses: A4_HUMAN (P05067), A4_MACFA (P53601), A4_MACMU (P29216), A4_MOUSE (P12023), A4_RAT (P08592), A4_SAISC (Q95241), AMBP_PLEPL (P36992), APP2_HUMAN (Q06481), APP2_RAT (P15943), AXP1_ANTAF (P81547), AXP2_ANTAF (P81548), BPT1_BOVIN (P00974), BPT2_BOVIN (P04815), CA17_HUMAN (Q02388), CA36_CHICK (P15989), CA36_HUMAN (P12111), CRPT_BOOMI (P81162), ELAC_MACEU (062845), ELAC_TRIVU (Q29143), EPPI_HUMAN (095925), EPPI_MOUSE (Q9DA01), HTIB_MANSE (P26227), IBP_CARCR (P00993), IBPC_BOVIN (P00976), IBPI_TACTR (P16044), IBPS_BOVIN (P00975), ICS3_BOMMO (P07481), IMAP_DROFU (P11424), IP52_ANESU (P10280), ISC1_BOMMO (P10831), ISC2_BOMMO (P10832), ISH1_STOHE (P31713), ISH2_STOHE (P81129), ISIK_HELPO (P00994), ISP2_GALME (P81906), IVB1_BUNFA(P25660), IVB1_BUNMU (P00987), IVB1-VIPAA (P00991), IVB2_BUNMU (P00989), IVB2_DABRU (P00990), IVB2_HEMHA (P00985), IVB2_NAJNI (P00986), IVB3_VIPAA (P00992), IVBB_DENPO (P00983), IVBC_NAJNA (P19859), IVBC_OPHHA (P82966), IVBE_DENPO (P00984), IVBI_DENAN (P00980), IVBI_DENPO (P00979), IVBK_DENAN (P00982), IVBK_DENPO (P00981), IVBT_ERIMA (P24541), IVBT_NAJNA (P20229), MCPI_MELCP (P82968), SBPI_SARBU (P26228), SPT3_HUMAN (P49223), TKD1_BOVIN (Q28201), TKD1_SHEEP (Q29428), TXCA_DENAN (P81658), UPTI_PIG (Q29100), AMBP_BOVIN (P00978), AMBP_HUMAN (P02760), AMBP_MERUN (Q62577), AMBP_MESAU (Q60559), AMBP_MOUSE (Q07456), AMBP_PIG (P04366), AMBP_RAT (Q64240), IATR_HORSE (P04365), IATR_SHEEP (P13371), SPT1_HUMAN (043278), SPT1_MOUSE (Q9R097), SPT2_HUMAN (043291), SPT2_MOUSE (Q9WU03), TFP2_HUMAN (P48307), TFP2_MOUSE (035536), TFPI_HUMAN (P10646), TFPI_MACMU (Q28864), TFPI_MOUSE (054819), TFPI_RABIT (P19761), TFPI_RAT (Q02445), and YN81_CAEEL (Q03610).

A "Kunitz conserved residue" at a particular amino acid position is an amino acid that is present, at that position, in at least 5 sequences of the foregoing list. A "Kunitz conserved residue" at a particular amino acid position is an amino acid that is present, at that position, in at least 30% of the sequences of the foregoing list. More than one Kunitze conserved or highly conserved residue may be available at a particular position. Positions are based on the optimal CLUSTALW alignment of the foregoing list, and are referenced accordingly to LACI-K1 amino acid numbering:

A variety of methods can be used to identify a Kunitz domain from a sequence database. For example, a known amino acid sequence of a Kunitz domain, a consensus sequence, or a motif (e.g., the ProSite Motif) can be searched against the GENBANK® sequence databases (National Center for Biotechnology Information, National Institutes of Health, Bethesda MD), e.g., using BLAST; against Pfam database of HMMs (Hidden Markov Models) (e.g., using default parameters for Pfam searching; against the SMART™ database; or against the ProDom database. See, e.g., Sonhammer et al. (1997) Proteins 28(3):405-420; Gribskov et al. (1990) Meth. Enzymol. 183:146-159; Gribskov et al. (1987) Proc. Natl. Acad. Sci. USA 84:4355-4358; Krogh et al. (1994) J. Mol. Biol. 235:1501-1531; Stultz et al. (1993) Protein Sci. 2:305-314; Schultz et al. (1998), Proc. Natl. Acad. Sci. USA 95:5857 Schultz et al. (2000) Nucl. Acids Res 28:231; and Corpet et al. (1999), Nucl. Acids Res. 27:263-267). Prosite lists the Kunitz domain as a motif and identifies proteins that include a Kunitz domain. See, e.g., Falquet et al. Nucleic Acids Res. 30:235-238(2002).

### Varying Kunitz Domains

Display libraries include variation at one or more positions in the displayed Kunitz domain. For example, between one and 20 or 5 and 12 positions can be varied. the varied positions can be located in one of the two interaction loops of the Kunitz domain. The first "interaction loop" includes P1, P1', P2', P3', and P4' and other amino acid positions corresponding to amino acids 11 to 19 of LACI-K1. The second "interaction loop" includes amino acid positions corresponding to amino acids 32 to 40 of LACI-K1.

The library can include variation in one or both interaction loops.

The theoretical library size, e.g., the number of unique display proteins that can be encoded by a library can be large (e.g., between 10³ to10¹⁹, 10³ to 10¹⁵, 10⁵ to 10¹⁴, or 10⁷ to 10¹² different display proteins, and/or e.g., at least 10⁵, 10⁶, 10⁸, 10⁹, or 10¹⁰. The theoretical size refers to the total number of unique amino acid sequences that could be encoded by the library in its completely represented form, regardless of an actual implementation. Theoretical diversity is generally the product of the number of variations at each position. For example, the theoretical diversity of varying only two positions among all twenty amino acids is 20 x 20, or 400. A library with large diversity is very useful even though the actual library used can might only sample a small subset of the theoretical diversity.

However, libraries with limited diversity, e.g., less than 10¹⁵, 10¹⁴, 10¹³, 10¹², 10¹¹, 10¹⁰, or 10⁹ different proteins are also useful. If appropriately designed, such libraries may also include a higher fraction of folded proteins. Libraries with limited diversity also facilitate rigorous evaluation of a particular sequence space.

**Synthetic Diversity**. Libraries can include regions of diverse nucleic acid sequence that originate from artificially synthesized sequences. Synthetic amino acid sequences include variants of naturally occurring sequences, e.g., variants that are at least 30, 50, 70, 80, 90, 95, or 98% identical to a naturally occurring sequence.

Typically, the library is synthesized from one or more degenerate oligonucleotide populations that include a distribution of nucleotides at a plurality of selected positions. The inclusion of a given nucleotide is random with respect to the distribution. One example of a degenerate source of synthetic diversity is an oligonucleotide that includes NNN wherein N is any of the four nucleotides in equal proportion. The degenerate oligonucleotide also includes invariant positions that encode invariant amino acid positions of the template Kunitz domain.

Synthetic diversity can also be more constrained, e.g., to limit the number of codons in a nucleic acid sequence at a given trinucleotide to a distribution that is smaller than NNN. For example, such a distribution can be constructed using less than four nucleotides (e.g., three or two) at some positions of the codon. In addition, trinucleotide addition technology can be used to further constrain the distribution.

So-called "trinucleotide addition technology" is described, e.g., in Wells et al. (1985) Gene 34:315-323, U.S. Patent No. US 4,760,025 and 5,869,644. Oligonucleotides are synthesized on a solid phase support, one codon (i.e., trinucleotide) at a time. The support includes many functional groups for synthesis such that many oligonucleotides are synthesized in parallel. The support is first exposed to a solution containing a mixture of the set of codons for the first position. The unit is protected so additional units are not added. The solution containing the first mixture is washed away and the solid support is deprotected so a second mixture containing a set of codons for a second position can be added to the attached first unit. The process is iterated to sequentially assemble multiple codons. Trinucleotide addition technology enables the synthesis of a nucleic acid that at a given position can encoded a number of amino acids. The frequency of these amino acids can be regulated by the proportion of codons in the mixture. Further, the choice of amino acids at the given position is not restricted to quadrants of the codon table as is the case if mixtures of single nucleotides are added during the synthesis.

In some embodiments, variations in amino acid sequences in diverse regions can be limited, e.g., to subsets of amino acids having similar side chains. Examples of limited variations in amino acid sequences include, e.g., all amino acids except cysteine; amino acids with basic side chains (e.g., lysine, arginine, histidine); amino acids with acidic side chains (e.g., aspartic acid, glutamic acid); amino acids with uncharged polar side chains (e.g., glycine, asparagine, glutamine, serine, threonine, tyrosine); amino acids with nonpolar side chains (e.g., alanine, valine, leucine, isoleucine, proline, phenylalanine, methionine, tryptophan); amino acids with aromatic side chains (e.g., tyrosine, phenylalanine, tryptophan, histidine). Variations at a particular position can also be limited to Kunitz domain conserved or highly conserved amino acid residues.

**Natural Diversity**. Libraries can include regions of diverse nucleic acid sequence that originate (or are synthesized based on) from different naturally-occurring sequences, e.g., different naturally occurring Kunitz domains. For some libraries, both synthetic and natural diversity are included.

**Mutagenesis**. In one embodiment, display library technology is used in an iterative mode. A first display library is used to identify one or more ligands for a target. These identified ligands are then varied using a mutagenesis method to form a second display library. Higher affinity ligands are then selected from the second library, e.g., by using higher stringency or more competitive binding and washing conditions.

In some implementations, the mutagenesis of a Kunitz domain is targeted to regions known or likely to be at the binding interface, e.g., one or more positions described herein, e.g., one or more positions in the interaction loops. Further, mutagenesis can be directed to framework regions near or adjacent to the interaction loops. In the case of Kunitz domains, mutagenesis can also be limited to one or two of the interaction loops, one or two amino acid positions therein. Focused mutagenesis can facilitate precise step-wise improvements.

Some exemplary mutagenesis techniques include: error-prone PCR (Leung et al. (1989) Technique 1:11-15), recombination, DNA shuffling using random cleavage (Stemmer (1994) Nature 389-391; termed "nucleic acid shuffling"), RACHITT™ (Coco et al. (2001) Nature Biotech. 19:354), site-directed mutagenesis (Zooler et al. (1987) Nucl Acids Res 10:6487-6504), cassette mutagenesis (Reidhaar-Olson (1991) Methods Enzymol. 208:564-586) and incorporation of degenerate oligonucleotides (Griffiths et al. (1994) EMBO J 13:3245). Mutagenesis can also be used to prepare an initial library of varied Kunitz domains.

In one example of iterative selection, the methods described herein are used to first identify a protein ligand from a display library that binds a target compound with at least a minimal binding specificity for a target or a minimal activity, e.g., an equilibrium dissociation constant for binding of less than 100 nM, 10 nM, or 1 nM. The nucleic acid sequence encoding the initial identified protein ligand is used as a template nucleic acid for the introduction of variations, e.g., to identify a second protein ligand that has enhanced properties (e.g., binding affinity, kinetics, or stability) relative to the initial protein ligand.

### Phage Display

Phage display utilizes bacteriophages to display varied polypeptides. The display protein can be linked to a bacteriophage coat protein with covalent, non-covalent, and non-peptide bonds. See, e.g., U.S. Patent No. 5,223,409, Crameri et al. (1993) Gene 137:69 and WO 01/05950. The linkage can result from translation of a nucleic acid encoding the varied component fused to the coat protein. The linkage can include a flexible peptide linker, a protease site, or an amino acid incorporated as a result of suppression of a stop codon.

Phage display is described, for example, in Ladner et al., U.S. Patent No. 5,223,409; Smith (1985) Science 228:1315-1317; WO 92/18619; WO 91/17271; WO 92/20791; WO 92/15679; WO 93/01288; WO 92/01047; WO 92/09690; WO 90/02809; de Haard et al. (1999) J. Biol. Chem 274:18218-30; Hoogenboom et al. (1998) Immunotechnology 4:1-20; Hoogenboom et al. (2000) Immunol Today 2:371-8; Fuchs et al. (1991) Bio/Technology 9:1370-1372; Hay et al. (1992) Hum Antibod Hybridomas 3:81-85; Huse et al. (1989) Science 246:1275-1281; Griffiths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrard et al. (1991) Biotechnology 9:1373-1377; Rebar et al. (1996) Methods Enzymol. 267:129-49; Hoogenboom et al. (1991) Nuc Acid Res 19:4133-4137; and Barbas et al. (1991) PNAS 88:7978-7982.

Phage display systems have been developed for Ff filamentous phage (phage fl, fd, and M13) as well as other bacteriophage (e.g. T7 bacteriophage and lambdoid phages; see, e.g., Santini (1998) J. Mol. Biol. 282:125-135; Rosenberg et al. (1996) Innovations 6:1-6; Houshmet al. (1999) Anal Biochem 268:363-370).

Nucleic acids suitable for phage display, e.g., phage vectors, have been described. See, e.g., Armstrong *et al.* (1996) Academic Press, Kay *et al.,* Ed. pp.35-53; Corey et al. (1993) Gene 128(1):129-34; Cwirla et al. (1990) Proc Natl Acad Sci USA 87(16):6378-82; Fowlkes et al. (1992) Biotechniques 13(3):422-8; Hoogenboom et al. (1991) Nucleic Acids Res 19(15):4133-7; McCafferty et al. (1990) Nature 348(6301):552-4; McConnell et al. (1994) Gene 151(1-2):115-8; Scott and Smith (1990) Science 249(4967):386-90.

**Phagemids**. An alternative configuration of phage display uses a phagemid vector. In a phagemid system, the nucleic acid encoding the display protein is provided on a plasmid, typically of length less than 6000 nucleotides. The plasmid includes a phage origin of replication so that the plasmid is incorporated into bacteriophage particles when bacterial cells bearing the plasmid are infected with helper phage, e.g. M13K01. Phagemids, however, lack a sufficient set of phage genes in order to produce stable phage particles. These phage genes can be provided by a helper phage. Typically, the helper phage provides an intact copy of gene III and other phage genes required for phage replication and assembly. Because he helper phage has a defective origin, the helper phage genome is not efficiently incorporated into phage particles relative to the plasmid that has a wild type origin. See, e.g., U.S. Pat. No. 5,821,047. The phagemid genome contains a selectable marker gene, *e.g. Amp^{R}* or *Kan^{R}* for the selection of cells that are infected by a member of the library.

**Phage Vectors**. Another configuration of phage display uses vectors that include a set of phage genes sufficient to produce an infectious phage particle when expressed, a phage packaging signal, and an autonomous replication sequence. For example, the vector can be a phage genome that has been modified to include a sequence encoding the display protein. Phage display vectors can further include a site into which a foreign nucleic acid sequence can be inserted, such as a multiple cloning site containing restriction enzyme digestion sites. Foreign nucleic acid sequences, e.g., that encode display proteins in phage vectors, can be linked to a ribosomal binding site, a signal sequence (e.g., a M13 signal sequence), and a transcriptional terminator sequence.

Vectors may be constructed by standard cloning techniques to contain sequence encoding a polypeptide that includes a Kunitz domain and a portion of a phage coat protein, and which is operably linked to a regulatable promoter. In some embodiments, a phage display vector includes two nucleic acid sequences that encode the same region of a phage coat protein. For example, the vector includes one sequence that encodes such a region in a position operably linked to the sequence encoding the display protein, and another sequence which encodes such a region in the context of the functional phage gene (e.g., a wild-type phage gene) that encodes the coat protein.

One advantage of phage vectors is that they do not require the use of helper phage, thus, simplifying library preparation, reducing possible library bias, and producing phage libraries free of particles that package helper phage nucleic acid. Phage display vectors can also include a selectable marker such as a drug resistance markers, e.g., an ampicillin resistance gene. However, unlike phagemids, it is also possible and sometimes advantageous to use phage vectors that do not include such a selectable marker.

### Coat proteins

Phage display systems typically utilize Ff filamentous phage. In implementations using filamentous phage, for example, the display protein is physically attached to a phage coat protein anchor domain. Co-expression of the display protein with another polypeptide having the same anchor domain, e.g., an endogenous copy of the coat protein, will result in competition for expression on the surface of the particle.

Phage coat proteins that can be used for protein display include (i) minor coat proteins of filamentous phage, such as gene III protein, and (ii) major coat proteins of filamentous phage such as gene VIII protein. Fusions to other phage coat proteins such as gene VI protein, gene VII protein, or gene IX protein can also be used (see, e.g., WO 00/71694).

Portions (e.g., domains or fragments) of these proteins may also be used. Useful portions include domains that are stably incorporated into the phage particle, e.g., so that the fusion protein remains in the particle throughout a selection procedure. In one embodiment, the anchor domain or "stump" domain of gene III protein used (see, e.g., U.S. 5,658,727 for a description of an exemplary gene III protein stump domain). As used herein, an "anchor domain" refers to a domain that is incorporated into a genetic package (e. g., a phage). A typical phage anchor domain is incorporated into the phage coat or capsid.

In another embodiment, the gene VIII protein is used. See, e.g., U.S. 5,223,409. The mature, full-length gene VIII protein can be linked to the display protein.

The filamentous phage display systems typically use protein fusions to physically attach the heterologous amino acid sequence to a phage coat protein or anchor domain. For example, the phage can include a gene that encodes a signal sequence, the heterologous amino acid sequence, and the anchor domain, e.g., a gene III protein anchor domain.

It is also possible to use other display formats to screen libraries of Kunitz domains, e.g., libraries whose variation is designed as described herein. Exemplary other display formats include cell-based display (e.g., yeast display) and nucleic acid-protein fusions. See, e.g., US 6,207,466 and WO 03/029456. Protein arrays can also be used. See, e.g., WO 01/40803, WO 99/51773, and US2002-0192673-A1.

### Promoters for Display Protein Expression

Regulatable promoters can be used to control the valency of the display protein. Regulated expression can be used to produce phage that have a low valency of the display protein. Many regulatable (e.g., inducible and/or repressible) promoter sequences are known. Such sequences include regulatable promoters whose activity can be altered or regulated by the intervention of user, e.g., by manipulation of an environmental parameter.

For example, an exogenous chemical compound can be added to regulate transcription of some promoters. Regulatable promoters can contain binding sites for one or m ore transcriptional activator or repressor protein. Synthetic promoters that include transcription factor binding sites can be constructed and can also be used as regulatable promoters.

Exemplary regulatable promoters include promoters responsive to an environmental parameter, e.g., thermal changes, hormones, metals, metabolites, antibiotics, or chemical agents. Regulatable promoters appropriate for use in *E*. *coli* include promoters which contain transcription factor binding sites from the *lac, tac, trp, trc,* and *tet* operator sequences, or operons, the alkaline phosphatase promoter (*pho*), an arabinose promoter such as an *araBAD* promoter, the rhamnose promoter, the promoters themselves, or functional fragments thereof (see, e.g., Elvin et al., 1990, Gene 37 : 123-126; Tabor and Richardson, 1998, Proc. Natl. Acad. Sci. U. S. A. 1074-1078; Chang et al., 1986, Gene 44 : 121-125; Lutz and Bujard, March 1997, Nucl. Acids. Res. 25: 1203-1210; D. V. Goeddel et al., Proc. Nat. Acad. Sci. U.S.A., 76:106-110, 1979; J. D. Windass et al. Nucl. Acids. Res., 10:6639-57, 1982; R. Crowl et al., Gene, 38:31-38, 1985; Brosius, 1984, Gene 27 : 161-172; Amanna and Brosius, 1985, Gene 40 : 183-190; Guzman et al.,1992, J. Bacteriol., 174: 7716-7728; Haldimann et al., 1998, J. Bacteriol., 180: 1277-1286). The *tac* promoter is an example of a synthetic promoter.

The *lac* promoter, for example, can be induced by lactose or structurally related molecules such as isopropyl-beta-D-thiogalactoside (IPTG) and is repressed by glucose. Some inducible promoters are induced by a process of derepression, e.g., inactivation of a repressor molecule.

A regulatable promoter sequence can also be indirectly regulated. Examples of promoters that can be engineered for indirect regulation include: the phage lambda P_{R} - P_{L}, phage T7, SP6, and T5 promoters. For example, the regulatory sequence is repressed or activated by a factor whose expression is regulated, e.g., by an environmental parameter. One example of such a promoter is a T7 promoter. The expression of the T7 RNApolymerase can be regulated by an environmentally-responsive promoter such as the *lac* promoter. For example, the cell can include a heterologous nucleic acid that includes a sequence encoding the T7 RNA polymerase and a regulatory sequence (e.g., the *lac* promoter) that is regulated by an environmental parameter. The activity of the T7 RNA polymerase can also be regulated by the presence of a natural inhibitor of RNA polymerase, such as T7 lysozyme.

In another configuration, the *lambda P_{L}* can be engineered to be regulated by an environmental parameter. For example, the cell can include a nucleic acid sequence that encodes a temperature sensitive variant of the lambda repressor. Raising cells to the non-permissive temperature releases the P_{L} promoter from repression.

The regulatory properties of a promoter or transcriptional regulatory sequence can be easily tested by operably linking the promoter or sequence to a sequence encoding a reporter protein (or any detectable protein). This construct is introduced into a bacterial cell and the abundance of the reporter protein is evaluated under a variety of environmental conditions. A useful promoter or sequence is one that is selectively activated or repressed in certain conditions.

In some embodiments, non-regulatable promoters are used. For example, a promoter can be selected that produces an appropriate amount of transcription under the relevant conditions. An example of a non-regulatable promoter is the gene III promoter.

In one embodiment, the promoters are arranged as described in 10/723,981.

### Phage production and screening

Phage display libraries can be used identify Kunitz domains that interact with a target, e.g., a target compound. In one embodiment, the method includes amplifying a phage library member recovered in a selection for binders of a target compound.

One exemplary method of screening and amplifying phage includes the following:
a. Contacting a plurality of diverse display phage to a target compound;
b. Separating phage that bind to the target compound from unbound phage;
c. Recovering phage that bound the target compound;
d. Infecting host cells with the phage that bound the target compound;
e. Producing replicate phage from the infected cells;
f. optionally, repeating a. to d. one or more times, e.g., one to six times;
g. Recovering the bound phage or the nucleic acid within the phage, e.g., for individual characterization.

The method can be adapted for use with either phage that contain phage genomes or phage that contain phagemids.

To produce the phage (e.g., in step e., or prior to step a.) host cells are maintained under conditions that provide a selected level of transcriptional activity of the regulatable, e.g., inducible, promoter during phage production. In an example in which the inducible promoter is a *lac* promoter, a *lac* inducer (e.g., IPTG), or an agent that inhibits activity of a *lac* promoter (e.g., glucose) can be included in the growth medium. In one embodiment, high concentrations of glucose (e.g., 1 %) are used. In another embodiment, low concentrations of glucose are used (e.g., <0.1 %).

Regulation of expression of a display protein, e.g., containing a Kunitz domain, can provide a means of regulation of valency of the domain on the surface of the phage particle. For implementations in which the Kunitz domain is expressed as a fusion with a portion of minor coat protein, e.g., the anchor domain of the gene III protein, is under the control of the *lac* promoter, and is co-expressed with another copy of the coat protein, e.g., a full-length gene III protein, the valency of the display protein can be varied as follows: In the presence of glucose, the *lac* promoter will be repressed, and the display protein will be expressed at a low valency, e.g., 0-1 copies per phage particle; in the presence of IPTQ the lac promoter will be induced, and the display protein will be expressed at a higher valency, e.g., at least 1.0,1.5,1.8, or 2.0 copies per phage particle, e.g., on average.

In some implementations, the display protein is linked to the major coat protein (VIII). Phage produce large amounts of gene VIII protein (VIII). Partial secretion of the display protein linked to mature VIII can be less than the production of wild type VIII. Therefore, reduced valency of the display protein can also be achieved by linking the display protein to VIII, e.g., mature VIII.

Conditions for phage production may include a change in temperature. Lowering the incubation temperature for a specified time interval during phage production can facilitate folding of the display amino acid sequence One exemplary procedure for culturing host cells during phage production includes a 20 minute incubation period at 37°C followed by a 25 minute incubation period at 30°C.

After any given cycle of selection, individual phage can be analyzed by isolating colonies of cells infected under low multiplicity of infection conditions. Each bacterial colony is cultured under conditions that result in production of phage, e.g., in microtiter wells. Phage are harvested from each culture and used in an ELISA assay. The target compound is bound to a well of microtiter plate and contacted with phage. The plates are washed and the amount of bound phage are detected, e.g., using an antibody to the phage.

Selection of phage that bind a target molecule includes contacting the phage to the target molecule. The target molecule can be bound to a solid support, either directly or indirectly. Phage particles that bind to the target are then immobilized and separated from members that do not bind the target. Conditions of the separating step can vary in stringency. For example, pH and ionic strength can be varied from physiological conditions. Multiple cycles of binding and separation can be performed.

Covalent and non-covalent methods can be used to attach target molecules to a solid or insoluble support. Such supports can include a matrix, bead, resin, planar surface, or immunotube. In one example of a non-covalent method of attachment, target molecules are attached to one member of a binding pair. The other member of the binding pair is attached to a support. Streptavidin and biotin are one example of a binding pair that interact with high affinity. Other non-covalent binding pairs include glutathione-S-transferase and glutathione (see, e.g., U.S. 5,654,176), hexa-histidine and Ni²⁺ (see, e.g., German Patent No. DE 19507 166), and an antibody and a peptide epitope (see, e.g., Kolodziej and Young (1991) Methods Enz. 194:508-519 for general methods of providing an epitope tag).

Covalent methods of attachment of target compounds include chemical crosslinking methods. Reactive reagents can create covalent bonds between functional groups on the target molecule and the support. Examples of functional groups that can be chemically reacted are amino, thiol, and carboxyl groups. N-ethylmaleimide, iodoacetamide, N-hydrosuccinimide, and glutaraldehyde are examples of reagents that react with functional groups.

Display library members can be selected or captured with a variety of methods. Phage can be captured by adherence to a vessel, such as a microtiter plate, that is coated with the target molecule. Alternatively, phage can contact target molecules that are immobilized within a flow chamber, such as a chromatography column. Phage particles can also be captured by magnetically responsive particles such as paramagnetic beads. The beads can be coated with a reagent that can bind the target compound (e.g., an antibody), or a reagent that can indirectly bind a target compound (e.g., streptavidin-coated beads binding to biotinylated target compounds).

The identification of useful members of display can be automated. See, e.g., US-2003-0129659-A1. Devices suitable for automation include multi-well plate conveyance systems, magnetic bead particle processors, liquid handling units, colony picking units, and other robotics. These devices can be built on custom specifications or purchased from commercial sources, such as Autogen (Framingham MA), Beckman Coulter (USA), Biorobotics (Woburn MA), Genetix (New Milton, Hampshire UK), Hamilton (Reno NV), Hudson (Springfield NJ), Labsystems (Helsinki, Finland), Packard Bioscience (Meriden CT), and Tecan (Mannedorf, Switzerland).

In some cases, the methods described herein include an automated process for handling magnetic particles. The target compound is immobilized on the magnetic particles. The KingFisher™ system, a magnetic particle processor from Thermo LabSystems (Helsinki, Finland), for example, can be used to select display library members against the target. The display library is contacted to the magnetic particles in a tube. The beads and library are mixed. Then a magnetic pin, covered by a disposable sheath, retrieves the magnetic particles and transfers them to another tube that includes a wash solution. The particles are mixed with the wash solution. In this manner, the magnetic particle processor can be used to serially transfer the magnetic particles to multiple tubes to wash non-specifically or weakly bound library members from the particles. After washing, the particles can be transferred to a vessel that includes a medium that supports display library member amplification. In the case of phage display the vessel may also include host cells.

In some cases, e.g., for phage display, the processor can also separate infected host cells from the previously-used particles. The processor can also add a new supply of magnetic particles for an additional round of selection.

The use of automation to perform the selection can increase the reproducibility of the selection process as well as the through-put.

An exemplary magnetically responsive particle is the DYNABEAD® available from DYNAL BIOTECH (Oslo, Norway). DYNABEADS® provide a spherical surface of uniform size, e.g., 2 m, 4.5 µm, and 5.0 µm diameter. The beads include gamma Fe₂O₃ and Fe₃O₄ as magnetic material. The particles are superparamagnetic as they have magnetic properties in a magnetic field, but lack residual magnetism outside the field. The particles are available with a variety of surfaces, e.g., hydrophilic with a carboxylated surface and hydrophobic with a tosyl-activated surface. Particles can also be blocked with a blocking agent, such as BSA or casein to reduce non-specific binding and coupling of compounds other than the target to the particle.

The target is attached to the paramagnetic particle directly or indirectly. A variety of target molecules can be purchased in a form linked to paramagnetic particles. In one example, a target is chemically coupled to a particle that includes a reactive group, e.g., a crosslinker (e.g., N-hydroxy-succinimidyl ester) or a thiol.

In another example, the target is linked to the particle using a member of a specific binding pair. For example, the target can be coupled to biotin. The target is then bound to paramagnetic particles that are coated with streptavidin (e.g., M-270 and M-280 Streptavidin DYNAPARTICLES® available from DYNAL BIOTECH, Oslo, Norway). In one embodiment, the target is contacted to the sample prior to attachment of the target to the paramagnetic particles.

In some implementations, automation is also used to analyze display library members identified in the selection process. From the final sample, individual clones of each display member can be obtained. The Kunitz domain of each member can be individually analyzed, e.g., to assess a functional property. For example, the domain can be evaluated to determine if it affects the enzymatic activity of a target protease in vitro or in vivo. Methods for evaluating protease activity and its kinetics are well known. For example, digestion of labeled substrates can be evaluated.

Exemplary functional properties include: a kinetic parameter (e.g., for binding to the target compound), an equilibrium parameter (e.g., avidity, affinity, and so forth, e.g., for binding to the target compound), a structural or biochemical property (e.g., thermal stability, oligomerization state, solubility and so forth), and a physiological property (e.g., renal clearance, toxicity, target tissue specificity, and so forth) and so forth. Methods for analyzing binding parameters include ELISA, homogenous binding assays, and SPR. For example, ELISAs on a displayed protein, e.g., containing a varied Kunitz domain, can be performed directly, e.g., in the context of the phage or other display vehicle, or the displayed protein removed from the context of the phage or other display vehicle.

Each member can also be sequenced, e.g., to determine the amino acid sequence of the Kunitz domain that is displayed.

### Target compounds

In one aspect, the method pertains to the selection of phage that bind a target molecule. Any compound can serve as a target molecule. The target molecule may be a small molecule (e.g., a small organic or inorganic molecule), a polypeptide, a nucleic acid, a polysaccharide, and so forth. By way of example, a number of examples and configurations are described for targets. Of course, target compounds other than, or having properties other than those listed below, can be used.

Kunitz domain libraries can be used to select polypeptides that are capable of inhibiting proteases. For example, the method herein can be used to identify Kunitz domains, particularly effectively human Kunitz domains, that bind and/or inhibit plasmin, trypsin, chymotrypsin, elastase, and other proteases.

Active and inactive forms of the protease can be used. For example, active forms include forms that have been activated from a zymogen, e.g., by removal of a pro-domain. Secreted proteases are typically also processed to remove a signal sequence.

Inactive forms include chemically modified proteases and genetically modified proteases. Still other inactive forms include proteases in a zymogen form and proteases that are bound by an inhibitor or other inactivating molecule. Genetically modified proteases can include genetic alterations (e.g., a substitution, insertion, or deletion) that decrease activity at least 20% (e.g., at least 30, 40, 50, 60, 70, 80, 90, 95, or 99%). An alteration can be in or near the active site, for example, at an active site residue, e.g., a member of a catalytic triad.

For example, genetically modified proteases can be used to provide an inactive forms in which the active site is not occluded. This molecule can be used, e.g., in an initial screen or selection, to find Kunitz domains that bind the active site, even if such domains are susceptible to cleavage by the target protease. Inactive forms in which the active site is occluded (e.g., by the binding of an inhibitor) can be used to discard Kunitz domains which interact with the target protease, but not at the active site.

Protein target molecules may have a specific physical conformation, e.g. a folded or unfolded form, or an active or inactive form. In one embodiment, the protein has more than one specific conformation. For example, prions can adopt more than one conformation. Either the native or the diseased conformation can be a desirable target, e.g., to isolate agents that stabilize the native conformation or that identify or target the diseased conformation.

In some embodiments, the protein target is associated with a disease, e.g., neoplastic, cardiovascular, neurological, inflammatory and pulmonary diseases and disorders.

### Pharmaceutical Compositions

In another aspect, the invention provides a composition that includes a Kunitz-domain containing protein that binds to a target e.g., a target cell or molecule (e.g., a target protein, e.g., a protease). The composition can be a pharmaceutically acceptable composition. For example, the Kunitz-domain containing protein can be formulated together with a pharmaceutically acceptable carrier. As used herein, "pharmaceutical compositions" encompass labeled diagnostic compositions (e.g., for in vivo imaging), as well as therapeutic compositions.

As used herein, "pharmaceutically acceptable carrier" includes any and all solvents, dispersion media, coatings, antibacterial and antifungal agents, isotonic and absorption delaying agents, and the like that are physiologically compatible. Preferably, the carrier is suitable for intravenous, intramuscular, subcutaneous, parenteral, spinal or epidermal administration (*e.g.*, by injection or infusion). Depending on the route of administration, the Kunitz domain-containing protein may be coated in a material to protect the compound from,the action of acids and other natural conditions that may inactivate it.

A "pharmaceutically acceptable salt" refers to a salt that retains the desired biological activity of the parent compound and does not impart any undesired toxicological effects (see *e.g*., Berge, S.M., et al. (1977) J. Pharm. Sci. 66:1-19). Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

The compositions that include a Kunitz-domain containing protein may be in a variety of forms. These include, for example, liquid, semi-solid and solid dosage forms, such as liquid solutions (e.g., injectable and infusible solutions), dispersions or suspensions, tablets, pills, powders, liposomes and suppositories. Typical compositions are in the form ofinjectable or infusible solutions, such as compositions similar to those used for administration of antibodies to humans. A common mode of administration is parenteral (*e.g*., intravenous, subcutaneous, intraperitoneal, intramuscular). In one embodiment, the Kunitz-domain containing protein is administered by intravenous infusion or injection. In another embodiment, the Kunitz-domain containing protein is administered by intramuscular or subcutaneous injection.

The composition can be formulated as a solution, microemulsion, dispersion, liposome, or other ordered structure suitable to high drug concentration. Sterile injectable solutions can be prepared by incorporating the Kunitz-domain containing protein in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. The composition can be prepared by a method that includes drying or dehydration (e.g., vacuum drying and freeze-drying), sterile-filtering, particle dispersion, and surfactant addition. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known. *See, e.g.,* Sustained and Controlled Release Drug Delivery Systems, J.R. Robinson, ed., Marcel Dekker, Inc., New York, 1978.

The Kunitz-domain containing protein can be administered by a variety of methods, e.g., intravenous injection or infusion. For example, for therapeutic applications, the Kunitz-domain containing protein can be administered by intravenous infusion at a rate of less than 30, 20, 10, 5, or 1 mg/min to reach a dose of about 1 to 100 mg/m² or 7 to 25 mg/m². The route and/or mode of administration will vary depending upon the desired results.

Pharmaceutical compositions can be administered by a medical device, e.g., needleless hypodermic injection devices, implants, implantable pumps (e.g., micro-infusion pumps), inhalers, and suppositories. See, e.g., U.S. Patent Nos. 5,399,163, 5,383,851, 5,312,335, 5,064,413, 4,941,880, 4,790,824, 4,596,556, 4,487,603, 4.,486,194, 4,447,233, 4,447,224, 4,439,196, and 4,475,196. An implantable micro-infusion pump, for example, can be used to dispense a composition at a controlled rate

Dosage regimens are adjusted to provide the optimum desired response (*e.g*., a therapeutic response). For example, a single bolus may be administered, several divided doses may be administered over time or the dose may be proportionally reduced or increased as indicated by the exigencies of the therapeutic situation. Parenteral compositions can be formulated in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

An exemplary, non-limiting range for a therapeutically or prophylactically effective amount of a Kunitz-domain containing protein is 0.1-20 mg/kg, 0.02-2 mg/kg, 0.1- 5 mg/kg, or 1-10 mg/kg. The Kunitz-domain containing protein can be administered by intravenous infusion at a rate of less than 20, 10, 5, 1, or 0.3 mg/min to reach a dose of about 1 to 100 mg/m², about 5 to 30 mg/m², or about 0.5 to 7 mg/m². Dosage values may vary with the type and severity of the condition to be alleviated. Specific dosage regimens can be adjusted over time.

Pharmaceutical compositions may include a therapeutically or prophylactically effective amount of a Kunitz-domain containing protein. Such amounts refer to an amount effective, at dosages and for periods of time necessary, to achieve the desired therapeutic or prophylactic result. A "therapeutically effective dosage" preferably modulates a measurable parameter or a symptom of a relevant disorder by at least about 20%, 40%, 60%, or 80% relative to untreated subjects.

The ability of a Kunitz-domain containing protein to modulate a disorder can be evaluated in an animal model system. For example, the ability of a Kunitz-domain containing protein to inhibit at least one symptom of cancer can be evaluated in an animal model that has xenografted human tumors. *In vitro* assays can also be used.

In one embodiment, the Kunitz-domain containing protein is conjugated to an agent, e.g., a cytotoxic drug, or radioisotope.

Also within the scope of the invention are kits comprising a Kunitz-domain containing protein and instructions for use, e.g., treatment, prophylactic, or diagnostic use. In one embodiment, the instructions for diagnostic applications include the use of a Kunitz-domain containing protein to detect a target, *in vitro,* e.g., in a sample, e.g., a biopsy or cells from a patient having a cancer or neoplastic disorder, or *in vivo.* In another embodiment, the instructions for therapeutic applications include suggested dosages and/or modes of administration in a patient with a cancer or neoplastic disorder. The kit can further contain a least one additional reagent, such as a diagnostic or therapeutic agent, e.g., a diagnostic or therapeutic agent as described herein, and/or one or more additional protein that includes a target-binding Kunitz domain, formulated as appropriate, in one or more separate pharmaceutical preparations.

### Treatments

Kunitz-domain containing proteins identified by the method described herein and/or detailed herein have therapeutic and prophylactic utilities. For example, these proteins can be administered to cells in culture, e.g. *in vitro* or *ex vivo,* or in a subject, e.g., *in vivo,* to treat, prevent, and/or diagnose a variety of disorders, such as cancers and cardio-vascular disease.

As used herein, the term "treat" or "treatment" is defined as the application or administration of a Kunitz-domain containing protein to a subject or a cell or tissue of the subject to prevent, ameliorate, or cure the disorder or at least one symptom of the disorder. For example, the protein can be administered in an amount effective to ameliorate at least one symptom of a disorder.. As used herein, the term "subject includes human, e.g., a patient having the disorder, and non-human animals.

Kunitz domain-containing protein can be administered to a human subject, e.g., for therapeutic purposes, or to a non-human subject, e.g., for veterinary purposes or as an animal model of human disease.

The method can be used to treat a cancer including all types of cancerous growths or oncogenic processes, metastatic tissues or malignantly transformed cells, tissues, or organs, irrespective of histopathologic type or stage of invasiveness. Examples of cancerous disorders include, but are not limited to, solid tumors, soft tissue tumors, and metastatic lesions, and cancers of hematopoietic origin. Examples of solid tumors include malignancies, e.g., sarcomas, adenocarcinomas, and carcinomas, of the various organ systems, such as those affecting lung, breast, lymphoid, gastrointestinal (e.g., colon), and genitourinary tract (e.g., renal, urothelial cells), pharynx, prostate, and ovary.

The method can be used to treat a disorder characterized by excessive activity of a protease that the Kunitz domain-containing protein inhibits. For example, the protease may be a protease that can modify a clotting factor or an extracellular matrix component.

### Diagnostic Uses

Kunitz domain-containing proteins also have *in vitro* and *in vivo* diagnostic utilities. They can be used, e.g., in a diagnostic method for detecting the presence of a target, *in vitro* (e.g., a biological sample, such as tissue, biopsy, e.g., a cancerous tissue) or *in vivo* (e.g., *in vivo* imaging in a subject).

The method includes: (i) contacting a sample with the Kunitz domain-containing protein; and (ii) detecting formation of a complex between the Kunitz domain-containing protein and the sample. The method can also include contacting a reference sample (e.g., a control sample) with the Kunitz domain-containing protein, and determining the extent of formation of the complex between the Kunitz domain-containing protein an the sample relative to the same for the reference sample. A change, e.g., a statistically significant change, in the formation of the complex in the sample or subject relative to the control sample or subject can be indicative of the presence of a target in the sample.

Another method includes: (i) administering the Kunitz domain-containing protein to a subject; and (iii) detecting formation of a complex between the Kunitz domain-containing protein and the subject. The detecting can include determining location or time of formation of the complex.

The Kunitz domain-containing protein can be directly or indirectly labeled with a detectable substance to facilitate detection. Suitable detectable substances include various enzymes, prosthetic groups, fluorescent materials, luminescent materials and radioactive materials. Exemplary fluorescent molecules include xanthene dyes, e.g., fluorescein and rhodamine, and naphthylamines. Examples of labels useful for diagnostic imaging in accordance with the present invention include radiolabels such as ¹³¹I, ¹¹¹In, ¹²³I, ^{99m}Tc, ³²P, ¹²⁵I, ³H, ¹⁴C, and ¹⁸⁸Rh, fluorescent labels such as fluorescein and rhodamine, nuclear magnetic resonance active labels, positron emitting isotopes detectable by a positron emission tomography ("PET") scanner, chemiluminescers such as luciferin, and enzymatic markers such as peroxidase or phosphatase. Examples of such contrast agents include paramagnetic agents and ferromagnetic or superparamagnetic agents. Chelates (e.g., EDTA, DTPA and NTA chelates) can be used to attach (and reduce toxicity) of some paramagnetic substances (e.g., Fe⁺³, Mn⁺², Gd⁺³). Kunitz domain-containing proteins can also be labeled with an indicating group containing of the NMR-active ¹⁹F atom.

### EXAMPLES

### Example 1: Generation of Kunitz domain library inserts.

The library was prepared using the oligonucleotides prepared using activated trinucleotide phosphoramidites for the variegated codons and normal single nucleotide addition for the constant regions. The amino acid sequence of this Kunitz domain library is depicted in FIG 4.

**Table 1: Design of a first exemplary library**

| **Position** | **Variability** | **Comment** |
|---|---|---|
| 11 | 19 | all except C |
| 13 | 19 | all except C |
| 15 | 18 | all except C and P |
| 16 | 6 | AGEDHT |
| 17 | 18 | all except C and P |
| 18 | 18 | all except C and P |
| 19 | 19 | all except C |
| | | |
| 34 | 19 | all except C |
| 39 | 19 | all except C |
| 40 | 2 | AG |
| | | |
| **Total** | **1.73E+11** | |

### Example 2: Generation of a first exemplary Kunitz domain library.

Following PCR assembly of the library insert, a restriction digest was performed using enzymes *Nco*I and *Xba*I*.* The library insert was purified and then ligated into the monovalent phage display vector DY3P82 which had been similarly digested and purified. The ligated DNA was transformed into electrocompetent DH5-α cells resulting in a total of 2.8 x 10⁹ transformants.

The display vector, DY3P82, is ampicillin resistant, contains a full length copy of the gene *iii* and also a truncated copy of the gene *iii* as anchor for the displayed Kunitz domain (Fig. 1). Expression of the display/gene III fusion is controlled by the *lac* promoter/operator. Use of the *lac* promoter allows control of the level of expression and consequently the level of display by the addition of IPTG (induction of display) or glucose (repression of display).

Phage DY3P82 is a derivative of M13mp18. DY3P82 was constructed by changing the KasI site of M13mp18 into a BamHI site. The segment from this BamHI site to the Bsu36I site was replaced with the DNA shown. This comprises a *bla* gene (obtained from pGemZ3f and modified by removal of the ApaLI and BssSI restriction sites) and the display cassette. This exemplary display cassette includes: a) the PlacZ promoter (between XhoI and PflMI), b) a ribosome binding site upstream of the M13 signal sequence, c) a modified M13 signal sequence that contains NcoI and EagI restriction sites, d) parts of the LACI-K1 domain including NsiI, MluI, AgeI, and XbaI sites, e) a linker including an NheI site, f) the third domain of M13 gene III (the DNA encodes the amino acid sequence of M13 domain 3, but many of the codons are picked to be different from those of endogenous gene III), g) two stop codons, h) an AvrII site i) the trp terminator, and j) NsiI site (of which there are two in the vector).

### Example 3: Display by a Kunitz domain library.

Phage containing the library were prepared either in the presence of glucose (no display) or in the presence of IPTG (display) and an ELISA performed using an anti-DX-88 polyclonal antibody preparation. This library also had improved handling properties, e.g., an improved viscosity.

DX-88 is a LACI-K1 derived Kunitz domain. Anti-DX-88 polyclonal antibodies cross react with isolates from LACI-K1 libraries in both western blots and ELISAs.

Wells of a microtitre plate were coated with an anti-gene VIII antibody to facilitate capture of the phage. The wells were then blocked with an appropriate reagent (such as BSA) to prevent non-specific binding and finally washed with PBS/0.05% Tween-20 (PBST). The library phage were then applied to the microtitre plate and allowed to bind for 1 hour at 37°C. Non-bound phage were washed away, using PBST, prior to addition of an anti-DX-88 antibody. The DX-88 antibody was allowed to bind, then washed with PBST and an appropriate secondary antibody conjugated to HRP added. Following incubation with the secondary antibody and washing, the ELISA was developed using TMB.

Phage that display DX-88 polyvalently were included as a positive control. These phage are not regulatable with IPTG or glucose. Shifting from induction conditions (plus IPTG) to repression conditions (plus glucose) should have no effect on the DX-88 phage. Clones R1F4, R2D1, R2F6, R2F8 are antibody isolates cloned into the DY3P82 phage vector and are included as negative controls.

### Example 4: Identification of serine protease inhibitors with a Kunitz domain library.

The monovalent Kunitz library has been used to identify binders (and presumably inhibitors) to a recombinant serine protease (rSerProtease-1). Three rounds of selection were performed. Binding of the phage to the biotinylated rSerProtease-1 target was in solution for two hours followed by capture of the phage-target complex on streptavidin coated magnetic beads. ELISA analysis of phage isolates from the third round was performed using rSerProtease-1 coated plates and an anti-geneVIII antibody to detect the phage. We isolated a number of phage that specifically bind to rSerProtease-1.

### Example 5: A second exemplary Kunitz domain library

A Kunitz domain library similar to the Kunitz domain library in Example 4 was also constructed. The amino acid sequence of this library is shown in FIG 5. This library contains additional sites of variation at positions 32, 34, 39, and 40. The theoretical size of this library is 2.64 x 10¹⁴ unique amino acid sequences.

In one embodiment, the library uses gene *iii* coat protein as anchor. The Kunitz domains are displayed at about five copies per phage. Although proteolysis in the periplasm may reduce the valency, each phage has two or more copies which can lead to unwanted avidity effects.

**Table 2: Design of second exemplary library**

| **Position** | **Variability** | **Comment** |
|---|---|---|
| 11 | 19 | all except C |
| 13 | 19 | all except C |
| 15 | 18 | all except C and P |
| 16 | 6 | AGEDHT |
| 17 | 18 | all except C and P |
| 18 | 18 | all except C and P |
| 19 | 19 | all except C |
| 32 | 19 | all except C |
| 34 | 19 | all except C |
| 39 | 19 | all except C |
| 40 | 18 | all except C and P |
| 46 | 18 | all except C and P |
| | | |
| **Total** | 5.33E+14 | |

### Example 6

The following are exemplary vector sequences.
**DY3P82** 
AATGCTACTACTATTAGTAGAATTGATGCCACCTTTTCAGCTCGCGCCCCAAATGAAA
ATATAGCTAAACAGGTTATTGACCATTTGCGAAATGTATCTAATGGTCAAACTAAATC
TACTCGTTCGCAGAATTGGGAATCAACTGTTATATGGAATGAAACTTCCAGACACCGT
ACTTTAGTTGCATATTTAAAACATGTTGAGCTACAGCATTATATTCAGCAATTAAGCT
CTAAGCCATCCGCAAAAATGACCTCTTATCAAAAGGAGCAATTAAAGGTACTCTCTAA
TCCTGACCTGTTGGAGTTTGCTTCCGGTCTGGTTCGCTTTGAAGCTCGAATTAAAACG
CGATATTTGAAGTCTTTCGGGCTTCCTCTTAATCTTTTTGATGCAATCCGCTTTGCTT
CTGACTATAATAGTCAGGGTAAAGACCTGATTTTTGATTTATGGTCATTCTCGTTTTC
TGAACTGTTTAAAGCATTTGAGGGGGATTCAATGAATATTTATGACGATTCCGCAGTA
TTGGACGCTATCCAGTCTAAACATTTTACTATTACCCCCTCTGGCAAAACTTCTTTTG
CAAAAGCCTCTCGCTATTTTGGTTTTTATCGTCGTCTGGTAAACGAGGGTTATGATAG
TGTTGCTCTTACTATGCCTCGTAATTCCTTTTGGCGTTATGTATCTGCATTAGTTGAA
TGTGGTATTCCTAAATCTCAACTGATGAATCTTTCTACCTGTAATAATGTTGTTCCGT
TAGTTCGTTTTATTAACGTAGATTTTTCTTCCCAACGTCCTGACTGGTATAATGAGCC
AGTTCTTAAAATCGCATAAGGTAATTCACAATGATTAAAGTTGAAATTAAACCATCTC
AAGCCCAATTTACTACTCGTTCTGGTGTTTCTCGTCAGGGCAAGCCTTATTCACTGAA
TGAGCAGCTTTGTTACGTTGATTTGGGTAATGAATATCCGGTTCTTGTCAAGATTACT
CTTGATGAAGGTCAGCCAGCCTATGCGCCTGGTCTGTACACCGTTCATCTGTCCTCTT
TCAAAGTTGGTCAGTTCGGTTCCCTTATGATTGACCGTCTGCGCCTCGTTCCGGCTAA
GTAACATGGAGCAGGTCGCGGATTTCGACACAATTTATCAGGCGATGATACAAATCTC
CGTTGTACTTTGTTTCGCGCTTGGTATAATCGCTGGGGGTCAAAGATGAGTGTTTTAG
TGTATTCTTTTGCCTCTTTCGTTTTAGGTTGGTGCCTTCGTAGTGGCATTACGTATTT
TACCCGTTTAATGGAAACTTCCTCATGAAAAAGTCTTTAGTCCTCAAAGCCTCTGTAG
CCGTTGCTACCCTCGTTCCGATGCTGTCTTTCGCTGCTGAGGGTGACGATCCCGCAAA
 
AGCGGCCTTTAACTCCCTGCAAGCCTCAGCGACCGAATATATCGGTTATGCGTGGGCG
ATGGTTGTTGTCATTGTCGGCGCAACTATCGGTATCAAGCTGTTTAAGAAATTCACCT
CGAAAGCAAGCTGATAAACCGATACAATTAAAGGCTCCTTTTGGAGCCTTTTTTTTG
GAGATTTTCAACGTGAAAAAATTATTATTCGCAATTCCTTTAGTTGTTCCTTTCTATT
CTCACTCCGCTGAAACTGTTGAAAGTTGTTTAGCAAAATCCCATACAGAAAATTCATT
TACTAACGTCTGGAAAGACGACAAAACTTTAGATCGTTACGCTAACTATGAGGGCTGT
CTGTGGAATGCTACAGGCGTTGTAGTTTGTACTGGTGACGAAACTCAGTGTTACGGTA
CATGGGTTCCTATTGGGCTTGCTATCCCTGAAAATGAGGGTGGTGGCTCTGAGGGTGG
CGGTTCTGAGGGTGGCGGTTCTGAGGGTGGCGGTACTAAACCTCCTGAGTACGGTGAT
ACACCTATTCCGGGCTATACTTATATCAACCCTCTCGACGGCACTTATCCGCCTGGTA
CTGAGCAAAACCCCGCTAATCCTAATCCTTCTCTTGAGGAGTCTCAGCCTCTTAATAC
TTTCATGTTTCAGAATAATAGGTTCCGAAATAGGCAGGGGGCATTAACTGTTTATACG
GGCACTGTTACTCAAGGCACTGACCCCGTTAAAACTTATTACCAGTACACTCCTGTAT
CATCAAAAGCCATGTATGACGCTTACTGGAACGGTAAATTCAGAGACTGCGCTTTCCA
TTCTGGCTTTAATGAGGATTTATTTGTTTGTGAATATCAAGGCCAATCGTCTGACCTG
CCTCAACCTCCTGTCAATGCTGGCGGCGGCTCTGGTGGTGGTTCTGGTGGCGGCTCTG
AGGGTGGTGGCTCTGAGGGTGGCGGTTCTGAGGGTGGCGGCTCTGAGGGAGGCGGTTC
CGGTGGTGGCTCTGGTTCCGGTGATTTTGATTATGAAAAGATGGCAAACGCTAATAAG
GGGGCTATGACCGAAAATGCCGATGAAAACGCGCTACAGTCTGACGCTAAAGGCAAAC
TTGATTCTGTCGCTACTGATTACGGTGCTGCTATCGATGGTTTCATTGGTGACGTTTC
CGGCCTTGCTAATGGTAATGGTGCTACTGGTGATTTTGCTGGCTCTAATTCCCAAATG
GCTCAAGTCGGTGACGGTGATAATTCACCTTTAATGAATAATTTCCGTCAATATTTAC
CTTCCCTCCCTCAATCGGTTGAATGTCGCCCTTTTGTCTTTGGCGCTGGTAAACCATA
TGAATTTTCTATTGATTGTGACAAAATAAACTTATTCCGTGGTGTCTTTGCGTTTCTT
TTATATGTTGCCACCTTTATGTATGTATTTTCTACGTTTGCTAACATACTGCGTAATA
AGGAGTCTTAATCATGCCAGTTCTTTTGGGTATTCCGTTATTATTGCGTTTCCTCGGT
TTCCTTCTGGTAACTTTGTTCGGCTATCTGCTTACTTTTCTTAAAAAGGGCTTCGGTA
AGATAGCTATTGCTATTTCATTGTTTCTTGCTCTTATTATTGGGCTTAACTCAATTCT
TGTGGGTTATCTCTCTGATATTAGCGCTCAATTACCCTCTGACTTTGTTCAGGGTGTT
CAGTTAATTCTCCCGTCTAATGCGCTTCCCTGTTTTTATGTTATTCTCTCTGTAAAGG
CTGCTATTTTCATTTTTGACGTTAAACAAAAAATCGTTTCTTATTTGGATTGGGATAA
ATAATATGGCTGTTTATTTTGTAACTGGCAAATTAGGCTCTGGAAAGACGCTCGTTAG
CGTTGGTAAGATTCAGGATAAAATTGTAGCTGGGTGCAAAATAGCAACTAATCTTGAT
TTAAGGCTTCAAAACCTCCCGCAAGTCGGGAGGTTCGCTAAAACGCCTCGCGTTCTTA
GAATACCGGATAAGCCTTCTATATCTGATTTGCTTGCTATTGGGCGCGGTAATGATTC
CTACGATGAAAATAAAAACGGCTTGCTTGTTCTCGATGAGTGCGGTACTTGGTTTAAT
ACCCGTTCTTGGAATGATAAGGAAAGACAGCCGATTATTGATTGGTTTCTACATGCTC
GTAAATTAGGATGGGATATTATTTTTCTTGTTCAGGACTTATCTATTGTTGATAAACA
GGCGCGTTCTGCATTAGCTGAACATGTTGTTTATTGTCGTCGTCTGGACAGAATTACT
TTACCTTTTGTCGGTACTTTATATTCTCTTATTACTGGCTCGAAAATGCCTCTGCCTA
AATTACATGTTGGCGTTGTTAAATATGGCGATTCTCAATTAAGCCCTACTGTTGAGCG
TTGGCTTTATACTGGTAAGAATTTGTATAACGCATATGATACTAAACAGGCTTTTTCT
AGTAATTATGATTCCGGTGTTTATTCTTATTTAACGCCTTATTTATCACACGGTCGGT
ATTTCAAACCATTAAATTTAGGTCAGAAGATGAAATTAACTAAAATATATTTGAAAAA
GTTTTCTCGCGTTCTTTGTCTTGCGATTGGATTTGCATCAGCATTTACATATAGTTAT
ATAACCCAACCTAAGCCGGAGGTTAAAAAGGTAGTCTCTCAGACCTATGATTTTGATA
AATTCACTATTGACTCTTCTCAGCGTCTTAATCTAAGCTATCGCTATGTTTTCAAGGA
TTCTAAGGGAAAATTAATTAATAGCGACGATTTACAGAAGCAAGGTTATTCACTCACA
TATATTGATTTATGTACTGTTTCCATTAAAAAAGGTAATTCAAATGAAATTGTTAAAT
GTAATTAATTTTGTTTTCTTGATGTTTGTTTCATCATCTTCTTTTGCTCAGGTAATTG
 
AAATGAATAATTCGCCTCTGCGCGATTTTGTAACTTGGTATTCAAAGCAATCAGGCGA
ATCCGTTATTGTTTCTCCCGATGTAAAAGGTACTGTTACTGTATATTCATCTGACGTT
AAACCTGAAAATCTACGCAATTTCTTTATTTCTGTTTTACGTGCAAATAATTTTGATA
TGGTAGGTTCTAACCCTTCCATAATTCAGAAGTATAATCCAAACAATCAGGATTATAT
TGATGAATTGCCATCATCTGATAATCAGGAATATGATGATAATTCCGCTCCTTCTGGT
GGTTTCTTTGTTCCGCAAAATGATAATGTTACTCAAACTTTTAAAATTAATAACGTTC
GGGCAAAGGATTTAATACGAGTTGTCGAATTGTTTGTAAAGTCTAATACTTCTAAATC
CTCAAATGTATTATCTATTGACGGCTCTAATCTATTAGTTGTTAGTGCTCCTAAAGAT
ATTTTAGATAACCTTCCTCAATTCCTTTCAACTGTTGATTTGCCAACTGACCAGATAT
TGATTGAGGGTTTGATATTTGAGGTTCAGCAAGGTGATGCTTTAGATTTTTCATTTGC
TGCTGGCTCTCAGCGTGGCACTGTTGCAGGCGGTGTTAATACTGACCGCCTCACCTCT
GTTTTATCTTCTGCTGGTGGTTCGTTCGGTATTTTTAATGGCGATGTTTTAGGGCTAT
CAGTTCGCGCATTAAAGACTAATAGCCATTCAAAAATATTGTCTGTGCCACGTATTCT
TACGCTTTCAGGTCAGAAGGGTTCTATCTCTGTTGGCCAGAATGTCCCTTTTATTACT
GGTCGTGTGACTGGTGAATCTGCCAATGTAAATAATCCATTTCAGACGATTGAGCGTC
AAAATGTAGGTATTTCCATGAGCGTTTTTCCTGTTGCAATGGCTGGCGGTAATATTGT
TCTGGATATTACCAGCAAGGCCGATAGTTTGAGTTCTTCTACTCAGGCAAGTGATGTT
ATTACTAATCAAAGAAGTATTGCTACAACGGTTAATTTGCGTGATGGACAGACTCTTT
TACTCGGTGGCCTCACTGATTATAAAAACACTTCTCAGGATTCTGGCGTACCGTTCCT
GTCTAAAATCCCTTTAATCGGCCTCCTGTTTAGCTCCCGCTCTGATTCTAACGAGGAA
AGCACGTTATACGTGCTCGTCAAAGCAACCATAGTACGCGCCCTGTAGCGGCGCATTA
AGCGCGGCGGGTGTGGTGGTTACGCGCAGCGTGACCGCTACACTTGCCAGCGCCCTAG
CGCCCGCTCCTTTCGCTTTCTTCCCTTCCTTTCTCGCCACGTTCGCCGGCTTTCCCCG
TCAAGCTCTAAATCGGGGGCTCCCTTTAGGGTTCCGATTTAGTGCTTTACGGCACCTC
GACCCCAAAAAACTTGATTTGGGTGATGGTTGGCCATCGCCCTGATAGACGGTTTTTC
GCCCTTTGACGTTGGAGTCCACGTTCTTTAATAGTGGACTCTTGTTCCAAACTGGAAC
AACACTCAACCCTATCTCGGGCTATTCTTTTGATTTATAAGGGATTTTGCCGATTTCG
GAACCACCATCAAACAGGATTTTCGCCTGCTGGGGCAAACCAGCGTGGACCGCTTGCT
GCAACTCTCTCAGGGCCAGGCGGTGAAGGGCAATCAGCTGTTGCCCGTCTCACTGGTG
AAAAGAAAAACCACCCTGGATCCAAGCTTGCAGGTGGCACTTTTCGGGGAAATGTGCG
CGGAACCCCTATTTGTTTATTTTTCTAAATACATTCAAATATGTATCCGCTCATGAGA
CAATAACCCTGATAAATGCTTCAATAATATTGAAAAAGGAAGAGTATGAGTATTCAAC
ATTTCCGTGTCGCCCTTATTCCCTTTTTTGCGGCATTTTGCCTTCCTGTTTTTGCTCA
CCCAGAAACGCTGGTGAAAGTAAAAGATGCTGAAGATCAGTTGGGCGCACTAGTGGGT
TACATCGAACTGGATCTCAACAGCGGTAAGATCCTTGAGAGTTTTCGCCCCGAAGAAC
GTTTTCCAATGATGAGCACTTTTAAAGTTCTGCTATGTGGCGCGGTATTATCCCGTAT
TGACGCCGGGCAAGAGCAACTCGGTCGCCGCATACACTATTCTCAGAATGACTTGGTT
GAGTACTCACCAGTCACAGAAAAGCATCTTACGGATGGCATGACAGTAAGAGAATTAT
GCAGTGCTGCCATAACCATGAGTGATAACACTGCGGCCAACTTACTTCTGACAACGAT
CGGAGGACCGAAGGAGCTAACCGCTTTTTTGCACAACATGGGGGATCATGTAACTCGC
CTTGATCGTTGGGAACCGGAGCTGAATGAAGCCATACCAAACGACGAGCGTGACACCA
CGATGCCTGTAGCAATGGCAACAACGTTGCGCAAACTATTAACTGGCGAACTACTTAC
TCTAGCTTCCCGGCAACAATTAATAGACTGGATGGAGGCGGATAAAGTTGCAGGACCA
CTTCTGCGCTCGGCCCTTCCGGCTGGCTGGTTTATTGCTGATAAATCTGGAGCCGGTG
AGCGTGGGTCTCGCGGTATCATTGCAGCACTGGGGCCAGATGGTAAGCCCTCCCGTAT
CGTAGTTATCTACACGACGGGGAGTCAGGCAACTATGGATGAACGAAATAGACAGATC
GCTGAGATAGGTGCCTCACTGATTAAGCATTGGTAACTGTCAGACCAAGTTTACTCAT
ATATACTTTAGATTGATTTAAAACTTCATTTTTAATTTAAAAGGATCTAGGTGAAGAT
CCTTTTTGATAATCTCATGACCAAAATCCCTTAACGTGAGTTTTCGTTCCACTGTACG
TAAGACCCCCAAGCTTGTCGACTGAATGGCGAATGGCGCTTTGCCTGGTTTCCGGCAC
 
CAGAAGCGGTGCCGGAAAGCTGGCTGGAGTGCGATCTTCCTGACGCTCGAGCGCAACG
CAATTAATGTGAGTTAGCTCACTCATTAGGCACCCCAGGCTTTACACTTTATGCTTCC
GGCTCGTATGTTGTGTGGAATTGTGAGCGGATAACAATTTCACACAGGAAACAGCTAT
GACCATGATTACGCCAAGCTTTGGAGCCTTTTTTTTGGAGATTTTCAACGTGAAAAAA
TTATTATTCGCAATTCCTTTAGTTGTTCCTTTCTATTCCATGGCGGCCGAGATGCATT
CATTCATTTTCACGCGTCAGTGCGAGGAAAACCGGTTCGAGTCTCTAGAGGAATGTAA
GAAGATGTGCACTCGTGATTCTGCTAGCTCTGCTAGTGGCGACTTCGACTACGAGAAA
ATGGCTAATGCCAACAAAGGCGCCATGACTGAGAACGCTGACGAGAATGCTTTGCAAA
GCGATGCCAAGGGTAAGTTAGACAGCGTCGCGACCGACTATGGCGCCGCCATCGACGG
CTTTATCGGCGATGTCAGTGGTTTGGCCAACGGCAACGGAGCCACCGGAGACTTCGCA
GGTTCGAATTCTCAGATGGCCCAGGTTGGAGATGGGGACAACAGTCCGCTTATGAACA
ACTTTAGACAGTACCTTCCGTCTCTTCCGCAGAGTGTCGAGTGCCGTCCATTCGTTTT
CGGTGCCGGCAAGCCTTACGAGTTCAGCATCGACTGCGATAAGATCAATCTTTTCCGC
GGCGTTTTCGCTTTCTTGCTATACGTCGCTACTTTCATGTACGTTTTCAGCACTTTCG
CCAATATTTTACGCAACAAAGAAAGCTAGTGATCTCCTAGGAAGCCCGCCTAATGAGC
GGGCTTTTTTTTTCTGGTATGCATCCTGAGGCCGATACTGTCGTCGTCCCCTCAAACT
GGCAGATGCACGGTTACGATGCGCCCATCTACACCAACGTGACCTATCCCATTACGGT
CAATCCGCCGTTTGTTCCCACGGAGAATCCGACGGGTTGTTACTCGCTCACATTTAAT
GTTGATGAAAGCTGGCTACAGGAAGGCCAGACGCGAATTATTTTTGATGGCGTTCCTA
TTGGTTAAAAAATGAGCTGATTTAACAAAAATTTAATGCGAATTTTAACAAAATATTA
ACGTTTACAATTTAAATATTTGCTTATACAATCTTCCTGTTTTTGGGGCTTTTCTGAT
TATCAACCGGGGTACATATGATTGACATGCTAGTTTTACGATTACCGTTCATCGATTC
TCTTGTTTGCTCCAGACTCTCAGGCAATGACCTGATAGCCTTTGTAGATCTCTCAAAA
ATAGCTACCCTCTCCGGCATTAATTTATCAGCTAGAACGGTTGAATATCATATTGATG
GTGATTTGACTGTCTCCGGCCTTTCTCACCCTTTTGAATCTTTACCTACACATTACTC
AGGCATTGCATTTAAAATATATGAGGGTTCTAAAAATTTTTATCCTTGCGTTGAAATA
AAGGCTTCTCCCGCAAAAGTATTACAGGGTCATAATGTTTTTGGTACAACCGATTTAG
CTTTATGCTCTGAGGCTTTATTGCTTAATTTTGCTAATTCTTTGCCTTGCCTGTATGA
TTTATTGGATGTT
 **DY3P82_LACIK1**

AATGCTACTACTATTAGTAGAATTGATGCCACCTTTTCAGCTCGCGCCCCAA
ATGAAAATATAGCTAAACAGGTTATTGACCATTTGCGAAATGTATCTAATG
GTCAAACTAAATCTACTCGTTCGCAGAATTGGGAATCAACTGTTATATGGA
ATGAAACTTCCAGACACCGTACTTTAGTTGCATATTTAAAACATGTTGAGCT
ACAGCATTATATTCAGCAATTAAGCTCTAAGCCATCCGCAAAAATGACCTC
TTATCAAAAGGAGCAATTAAAGGTACTCTCTAATCCTGACCTGTTGGAGTTT
GCTTCCGGTCTGGTTCGCTTTGAAGCTCGAATTAAAACGCGATATTTGAAGT
CTTTCGGGCTTCCTCTTAATCTTTTTGATGCAATCCGCTTTGCTTCTGACTAT
AATAGTCAGGGTAAAGACCTGATTTTTGATTTATGGTCATTCTCGTTTTCTG
AACTGTTTAAAGCATTTGAGGGGGATTCAATGAATATTTATGACGATTCCG
CAGTATTGGACGCTATCCAGTCTAAACATTTTACTATTACCCCCTCTGGCAA
AACTTCTTTTGCAAAAGCCTCTCGCTATTTTGGTTTTTATCGTCGTCTGGTAA
ACGAGGGTTATGATAGTGTTGCTCTTACTATGCCTCGTAATTCCTTTTGGCG
TTATGTATCTGCATTAGTTGAATGTGGTATTCCTAAATCTCAACTGATGAAT
CTTTCTACCTGTAATAATGTTGTTCCGTTAGTTCGTTTTATTAACGTAGATTT
TTCTTCCCAACGTCCTGACTGGTATAATGAGCCAGTTCTTAAAATCGCATAA
GGTAATTCACAATGATTAAAGTTGAAATTAAACCATCTCAAGCCCAATTTA

CTACTCGTTCTGGTGTTTCTCGTCAGGGCAAGCCTTATTCACTGAATGAGCA
GCTTTGTTACGTTGATTTGGGTAATGAATATCCGGTTCTTGTCAAGATTACT
CTTGATGAAGGTCAGCCAGCCTATGCGCCTGGTCTGTACACCGTTCATCTGT
CCTCTTTCAAAGTTGGTCAGTTCGGTTCCCTTATGATTGACCGTCTGCGCCT
CGTTCCGGCTAAGTAACATGGAGCAGGTCGCGGATTTCGACACAATTTATC
AGGCGATGATACAAATCTCCGTTGTACTTTGTTTCGCGCTTGGTATAATCGC
TGGGGGTCAAAGATGAGTGTTTTAGTGTATTCTTTTGCCTCTTTCGTTTTAG
GTTGGTGCCTTCGTAGTGGCATTACGTATTTTACCCGTTTAATGGAAACTTC
CTCATGAAAAAGTCTTTAGTCCTCAAAGCCTCTGTAGCCGTTGCTACCCTCG
TTCCGATGCTGTCTTTCGCTGCTGAGGGTGACGATCCCGCAAAAGCGGCCTT
TAACTCCCTGCAAGCCTCAGCGACCGAATATATCGGTTATGCGTGGGCGAT
GGTTGTTGTCATTGTCGGCGCAACTATCGGTATCAAGCTGTTTAAGAAATTC
ACCTCGAAAGCAAGCTGATAAACCGATACAATTAAAGGCTCCTTTTGGAGC
CTTTTTTTTTGGAGATTTTCAACGTGAAAAAATTATTATTCGCAATTCCTTTA
GTTGTTCCTTTCTATTCTCACTCCGCTGAAACTGTTGAAAGTTGTTTAGCAA
AATCCCATACAGAAAATTCATTTACTAACGTCTGGAAAGACGACAAAACTT
TAGATCGTTACGCTAACTATGAGGGCTGTCTGTGGAATGCTACAGGCGTTG
TAGTTTGTACTGGTGACGAAACTCAGTGTTACGGTACATGGGTTCCTATTGG
GCTTGCTATCCCTGAAAATGAGGGTGGTGGCTCTGAGGGTGGCGGTTCTGA
GGGTGGCGGTTCTGAGGGTGGCGGTACTAAACCTCCTGAGTACGGTGATAC
ACCTATTCCGGGCTATACTTATATCAACCCTCTCGACGGCACTTATCCGCCT
GGTACTGAGCAAAACCCCGCTAATCCTAATCCTTCTCTTGAGGAGTCTCAG
CCTCTTAATACTTTCATGTTTCAGAATAATAGGTTCCGAAATAGGCAGGGG
GCATTAACTGTTTATACGGGCACTGTTACTCAAGGCACTGACCCCGTTAAA
ACTTATTACCAGTACACTCCTGTATCATCAAAAGCCATGTATGACGCTTACT
GGAACGGTAAATTCAGAGACTGCGCTTTCCATTCTGGCTTTAATGAGGATTT
ATTTGTTTGTGAATATCAAGGCCAATCGTCTGACCTGCCTCAACCTCCTGTC
AATGCTGGCGGCGGCTCTGGTGGTGGTTCTGGTGGCGGCTCTGAGGGTGGT
GGCTCTGAGGGTGGCGGTTCTGAGGGTGGCGGCTCTGAGGGAGGCGGTTCC
GGTGGTGGCTCTGGTTCCGGTGATTTTGATTATGAAAAGATGGCAAACGCT
AATAAGGGGGCTATGACCGAAAATGCCGATGAAAACGCGCTACAGTCTGA
CGCTAAAGGCAAACTTGATTCTGTCGCTACTGATTACGGTGCTGCTATCGAT
GGTTTCATTGGTGACGTTTCCGGCCTTGCTAATGGTAATGGTGCTACTGGTG
ATTTTGCTGGCTCTAATTCCCAAATGGCTCAAGTCGGTGACGGTGATAATTC
ACCTTTAATGAATAATTTCCGTCAATATTTACCTTCCCTCCCTCAATCGGTTG
AATGTCGCCCTTTTGTCTTTGGCGCTGGTAAACCATATGAATTTTCTATTGA
TTGTGACAAAATAAACTTATTCCGTGGTGTCTTTGCGTTTCTTTTATATGTTG
CCACCTTTATGTATGTATTTTCTACGTTTGCTAACATACTGCGTAATAAGGA
GTCTTAATCATGCCAGTTCTTTTGGGTATTCCGTTATTATTGCGTTTCCTCGG
TTTCCTTCTGGTAACTTTGTTCGGCTATCTGCTTACTTTTCTTAAAAAGGGCT
TCGGTAAGATAGCTATTGCTATTTCATTGTTTCTTGCTCTTATTATTGGGCTT
AACTCAATTCTTGTGGGTTATCTCTCTGATATTAGCGCTCAATTACCCTCTG
ACTTTGTTCAGGGTGTTCAGTTAATTCTCCCGTCTAATGCGCTTCCCTGTTTT
TATGTTATTCTCTCTGTAAAGGCTGCTATTTTCATTTTTGACGTTAAACAAA
AAATCGTTTCTTATTTGGATTGGGATAAATAATATGGCTGTTTATTTTGTAA
CTGGCAAATTAGGCTCTGGAAAGACGCTCGTTAGCGTTGGTAAGATTCAGG
ATAAAATTGTAGCTGGGTGCAAAATAGCAACTAATCTTGATTTAAGGCTTC
AAAACCTCCCGCAAGTCGGGAGGTTCGCTAAAACGCCTCGCGTTCTTAGAA
TACCGGATAAGCCTTCTATATCTGATTTGCTTGCTATTGGGCGCGGTAATGA

TTCCTACGATGAAAATAAAAACGGCTTGCTTGTTCTCGATGAGTGCGGTACT
TGGTTTAATACCCGTTCTTGGAATGATAAGGAAAGACAGCCGATTATTGAT
TGGTTTCTACATGCTCGTAAATTAGGATGGGATATTATTTTTCTTGTTCAGG
ACTTATCTATTGTTGATAAACAGGCGCGTTCTGCATTAGCTGAACATGTTGT
TTATTGTCGTCGTCTGGACAGAATTACTTTACCTTTTGTCGGTACTTTATATT
CTCTTATTACTGGCTCGAAAATGCCTCTGCCTAAATTACATGTTGGCGTTGT
TAAATATGGCGATTCTCAATTAAGCCCTACTGTTGAGCGTTGGCTTTATACT
GGTAAGAATTTGTATAACGCATATGATACTAAACAGGCTTTTTCTAGTAATT
ATGATTCCGGTGTTTATTCTTATTTAACGCCTTATTTATCACACGGTCGGTAT
TTCAAACCATTAAATTTAGGTCAGAAGATGAAATTAACTAAAATATATTTG
AAAAAGTTTTCTCGCGTTCTTTGTCTTGCGATTGGATTTGCATCAGCATTTA
CATATAGTTATATAACCCAACCTAAGCCGGAGGTTAAAAAGGTAGTCTCTC
AGACCTATGATTTTGATAAATTCACTATTGACTCTTCTCAGCGTCTTAATCT
AAGCTATCGCTATGTTTTCAAGGATTCTAAGGGAAAATTAATTAATAGCGA
CGATTTACAGAAGCAAGGTTATTCACTCACATATATTGATTTATGTACTGTT
TCCATTAAAAAAGGTAATTCAAATGAAATTGTTAAATGTAATTAATTTTGTT
TTCTTGATGTTTGTTTCATCATCTTCTTTTGCTCAGGTAATTGAAATGAATAA
TTCGCCTCTGCGCGATTTTGTAACTTGGTATTCAAAGCAATCAGGCGAATCC
GTTATTGTTTCTCCCGATGTAAAAGGTACTGTTACTGTATATTCATCTGACG
TTAAACCTGAAAATCTACGCAATTTCTTTATTTCTGTTTTACGTGCAAATAA
TTTTGATATGGTAGGTTCTAACCCTTCCATAATTCAGAAGTATAATCCAAAC
AATCAGGATTATATTGATGAATTGCCATCATCTGATAATCAGGAATATGAT
GATAATTCCGCTCCTTCTGGTGGTTTCTTTGTTCCGCAAAATGATAATGTTA
CTCAAACTTTTAAAATTAATAACGTTCGGGCAAAGGATTTAATACGAGTTG
TCGAATTGTTTGTAAAGTCTAATACTTCTAAATCCTCAAATGTATTATCTAT
TGACGGCTCTAATCTATTAGTTGTTAGTGCTCCTAAAGATATTTTAGATAAC
CTTCCTCAATTCCTTTCAACTGTTGATTTGCCAACTGACCAGATATTGATTG
AGGGTTTGATATTTGAGGTTCAGCAAGGTGATGCTTTAGATTTTTCATTTGC
TGCTGGCTCTCAGCGTGGCACTGTTGCAGGCGGTGTTAATACTGACCGCCTC
ACCTCTGTTTTATCTTCTGCTGGTGGTTCGTTCGGTATTTTTAATGGCGATGT
TTTAGGGCTATCAGTTCGCGCATTAAAGACTAATAGCCATTCAAAAATATT
GTCTGTGCCACGTATTCTTACGCTTTCAGGTCAGAAGGGTTCTATCTCTGTT
GGCCAGAATGTCCCTTTTATTACTGGTCGTGTGACTGGTGAATCTGCCAATG
TAAATAATCCATTTCAGACGATTGAGCGTCAAAATGTAGGTATTTCCATGA
GCGTTTTTCCTGTTGCAATGGCTGGCGGTAATATTGTTCTGGATATTACCAG
CAAGGCCGATAGTTTGAGTTCTTCTACTCAGGCAAGTGATGTTATTACTAAT
CAAAGAAGTATTGCTACAACGGTTAATTTGCGTGATGGACAGACTCTTTTA
CTCGGTGGCCTCACTGATTATAAAAACACTTCTCAGGATTCTGGCGTACCGT
TCCTGTCTAAAATCCCTTTAATCGGCCTCCTGTTTAGCTCCCGCTCTGATTCT
AACGAGGAAAGCACGTTATACGTGCTCGTCAAAGCAACCATAGTACGCGCC
CTGTAGCGGCGCATTAAGCGCGGCGGGTGTGGTGGTTACGCGCAGCGTGAC
CGCTACACTTGCCAGCGCCCTAGCGCCCGCTCCTTTCGCTTTCTTCCCTTCCT
TTCTCGCCACGTTCGCCGGCTTTCCCCGTCAAGCTCTAAATCGGGGGCTCCC
TTTAGGGTTCCGATTTAGTGCTTTACGGCACCTCGACCCCAAAAAACTTGAT
TTGGGTGATGGTTGGCCATCGCCCTGATAGACGGTTTTTCGCCCTTTGACGT
TGGAGTCCACGTTCTTTAATAGTGGACTCTTGTTCCAAACTGGAACAACACT
CAACCCTATCTCGGGCTATTCTTTTGATTTATAAGGGATTTTGCCGATTTCG
GAACCACCATCAAACAGGATTTTCGCCTGCTGGGGCAAACCAGCGTGGACC
GCTTGCTGCAACTCTCTCAGGGCCAGGCGGTGAAGGGCAATCAGCTGTTGC

CCGTCTCACTGGTGAAAAGAAAAACCACCCTGGATCCAAGCTTGCAGGTGG
CACTTTTCGGGGAAATGTGCGCGGAACCCCTATTTGTTTATTTTTCTAAATA
CATTCAAATATGTATCCGCTCATGAGACAATAACCCTGATAAATGCTTCAAT
AATATTGAAAAAGGAAGAGTATGAGTATTCAACATTTCCGTGTCGCCCTTA
TTCCCTTTTTTGCGGCATTTTGCCTTCCTGTTTTTGCTCACCCAGAAACGCTG
GTGAAAGTAAAAGATGCTGAAGATCAGTTGGGCGCACTAGTGGGTTACATC
GAACTGGATCTCAACAGCGGTAAGATCCTTGAGAGTTTTCGCCCCGAAGAA
CGTTTTCCAATGATGAGCACTTTTAAAGTTCTGCTATGTGGCGCGGTATTAT
CCCGTATTGACGCCGGGCAAGAGCAACTCGGTCGCCGCATACACTATTCTC
AGAATGACTTGGTTGAGTACTCACCAGTCACAGAAAAGCATCTTACGGATG
GCATGACAGTAAGAGAATTATGCAGTGCTGCCATAACCATGAGTGATAACA
CTGCGGCCAACTTACTTCTGACAACGATCGGAGGACCGAAGGAGCTAACCG
CTTTTTTGCACAACATGGGGGATCATGTAACTCGCCTTGATCGTTGGGAACC
GGAGCTGAATGAAGCCATACCAAACGACGAGCGTGACACCACGATGCCTG
TAGCAATGGCAACAACGTTGCGCAAACTATTAACTGGCGAACTACTTACTC
TAGCTTCCCGGCAACAATTAATAGACTGGATGGAGGCGGATAAAGTTGCAG
GACCACTTCTGCGCTCGGCCCTTCCGGCTGGCTGGTTTATTGCTGATAAATC
TGGAGCCGGTGAGCGTGGGTCTCGCGGTATCATTGCAGCACTGGGGCCAGA
TGGTAAGCCCTCCCGTATCGTAGTTATCTACACGACGGGGAGTCAGGCAAC
TATGGATGAACGAAATAGACAGATCGCTGAGATAGGTGCCTCACTGATTAA
GCATTGGTAACTGTCAGACCAAGTTTACTCATATATACTTTAGATTGATTTA
AAACTTCATTTTTAATTTAAAAGGATCTAGGTGAAGATCCTTTTTGATAATC
TCATGACCAAAATCCCTTAACGTGAGTTTTCGTTCCACTGTACGTAAGACCC
CCAAGCTTGTCGACTGAATGGCGAATGGCGCTTTGCCTGGTTTCCGGCACC
AGAAGCGGTGCCGGAAAGCTGGCTGGAGTGCGATCTTCCTGACGCTCGAGC
GCAACGCAATTAATGTGAGTTAGCTCACTCATTAGGCACCCCAGGCTTTAC
ACTTTATGCTTCCGGCTCGTATGTTGTGTGGAATTGTGAGCGGATAACAATT
TCACACAGGAAACAGCTATGACCATGATTACGCCAAGCTTTGGAGCCTTTT
TTTTGGAGATTTTCAACGTGAAAAAATTATTATTCGCAATTCCTTTAGTTGT
TCCTTTCTATTCCATGGCGGCCGAGATGCATTCATTCTGCGCTTTCAAAGCT
GATGACGGTCCGTGTAAAGCTATCATGAAACGTTTCTTCTTCAACATTTTCA
CGCGTCAATGTGAAGAGTTCATTTACGGTGGTTGTGAAGGTAACCAGAACC
GGTTCGAATCTCTAGAGGAATGTAAGAAGATGTGCACTCGTGATTCTGCTA
GCTCTGCTAGTGGCGACTTCGACTACGAGAAAATGGCTAATGCCAACAAAG
GCGCCATGACTGAGAACGCTGACGAGAATGCTTTGCAAAGCGATGCCAAG
GGTAAGTTAGACAGCGTCGCGACCGACTATGGCGCCGCCATCGACGGCTTT
ATCGGCGATGTCAGTGGTTTGGCCAACGGCAACGGAGCCACCGGAGACTTC
GCAGGTTCGAATTCTCAGATGGCCCAGGTTGGAGATGGGGACAACAGTCCG
CTTATGAACAACTTTAGACAGTACCTTCCGTCTCTTCCGCAGAGTGTCGAGT
GCCGTCCATTCGTTTTCGGTGCCGGCAAGCCTTACGAGTTCAGCATCGACTG
CGATAAGATCAATCTTTTCCGCGGCGTTTTCGCTTTCTTGCTATACGTCGCT
ACTTTCATGTACGTTTTCAGCACTTTCGCCAATATTTTACGCAACAAAGAA
GCTAGTGATCTCCTAGGAAGCCCGCCTAATGAGCGGGCTTTTTTTTTCTGGT
ATGCATCCTGAGGCCGATACTGTCGTCGTCCCCTCAAACTGGCAGATGCAC
GGTTACGATGCGCCCATCTACACCAACGTGACCTATCCCATTACGGTCAAT
CCGCCGTTTGTTCCCACGGAGAATCCGACGGGTTGTTACTCGCTCACATTTA
ATGTTGATGAAAGCTGGCTACAGGAAGGCCAGACGCGAATTATTTTTGATG
GCGTTCCTATTGGTTAAAAAATGAGCTGATTTAACAAAAATTTAATGCGAA
TTTTAACAAAATATTAACGTTTACAATTTAAATATTTGCTTATACAATCTTC

CTGTTTTGGGGCTTTCTGATTATCAACCGGGGTACATATGATTGACATGC
TAGTTTTACGATTACCGTTCATCGATTCTCTTGTTTGCTCCAGACTCTCAGGC
AATGACCTGATAGCCTTTGTAGATCTCTCAAAAATAGCTACCCTCTCCGGCA
TTAATTTATCAGCTAGAACGGTTGAATATCATATTGATGGTGATTTGACTGT
CTCCGGCCTTTCTCACCCTTTTGAATCTTTACCTACACATTACTCAGGCATTG
CATTTAAAATATATGAGGGTTCTAAAAATTTTTATCCTTGCGTTGAAATAAA
GGCTTCTCCCGCAAAAGTATTACAGGGTCATAATGTTTTTGGTACAACCGAT
TTAGCTTTATGCTCTGAGGCTTTATTGCTTAATTTTGCTAATTCTTTGCCTTG
CCTGTATGATTTATTGGATGTT

A number of embodiments of the invention have been described. Nevertheless, it will be understood that various modifications may be made without departing from the spirit and scope of the invention. Accordingly, other embodiments are within the scope of the following claims.

The following pages 51 to 54 contain preferred embodiments. Accordingly, the term "claim" as used therein refers to such a "preferred embodiment".

1. A library comprising a plurality of filamentous phage particles, each phage particle of the plurality including (i) a display protein that comprises a Kunitz domain, (ii) a nucleic acid comprising (a) phage genes sufficient to produce an infectious phage particle and (b) a sequence encoding the display protein,
   wherein the Kunitz domain includes at least varied one amino acid position in each of two interaction loops, the positions in the respective loops being varied among the particles of the plurality, and wherein the average number of copies of the Kunitz domain per phage particles of the plurality is less than 2.0.
2. The library of claim 1 wherein the Kunitz domain is at least 85% identical to a human Kunitz domain at amino acid positions that are not varied.
3. The library of claim 1 wherein between 5 and 12 amino acid positions are varied among the particles of the plurality.
4. The library of claim 3 wherein at least amino acid positions corresponding amino acid positions 16, 17, 18, 19, 34, and 39 of human LACI-K1 are varied among the particles of the plurality.
5. The library of claim 4 wherein at least amino acid positions corresponding amino acid positions 11, 13, 15, 16, 17, 18, 19, 34, and 39 of human LACI-K1 are varied among the particles of the plurality.
6. The library of claim 3 wherein only amino acid positions corresponding amino acid positions 11, 13, 15, 16, 17, 18, 19, 34, 39, and 40 of human LACI-K1 are varied among the particles of the plurality.
7. The library of claim 1 wherein the amino acid position corresponding to amino acid position 40 is G or A, or is varied between G and A.
8. The library of claim 1 wherein the display protein comprises a functional domain of a minor coat protein fused to the Kunitz domain, and the phage genes comprise a gene that encodes the minor coat protein that is (i') not fused to a non-viral amino acid sequence of at least five amino acids or (ii') not fused to a varied amino acid sequence.
9. A library comprising a plurality of phage particles, each phage particle of the plurality including
   (i) a display protein that comprises a Kunitz domain and at least a portion of the gene III phage coat protein,
   (ii) a functional gene III phage coat protein, and
   (iii) a nucleic acid comprising (a) phage genes sufficient to produce an infectious phage particle and (b) a sequence encoding the display protein,
   wherein the Kunitz domain comprises a sequence that is at least 85% identical to
   MHSFCAFKADX₁₁GX₁₃CX₁₅X₁₆X₁₇X₁₈X₁₉RFFFNIFTRQCEEFX₃₄YGGCX₃₉X₄₀ NQNRFESLEECKKMCTRDGA, at positions other than X;
   X₁₁ is one of: A, D, E, F, G, H, I, K, L, M, N, P, Q, R, S, T, V, W, Y;
   X₁₃ is one of: A, D, E, F, G, H, I, K, L, M, N, P, Q, R, S, T, V, W, Y;
   X₁₅ is one of: A, D, E, F, G, H, I, K, L, M, N, Q, R, S, T, V, W, Y;
   X₁₆ is one of: A, G, E, D, H, T;
   X₁₇ is one of: A, D, E, F, G, H, I, K, L, M, N, Q, R, S, T, V, W, Y;
   X₁₈ is one of: A, D, E, F, G, H, I, K, L, M, N, Q, R, S, T, V, W, Y;
   X₁₉ is one of: A, D, E, F, G, H, I, K, L, M, N, P, Q, R, S, T, V, W, Y; X₃₄ is one of: A, C, D, E, F, G, H, I, K, L, M, N, P, Q, R, S, T, V, W, Y;
   X₃₉ is one of: A, C, D, E, F, G, H, I, K, L, M, N, P, Q, R, S, T, V, W, Y; and X₄₀ is one of: G, A, and
   at least two of X₁₁, X₁₃, X₁₅, X₁₆, X₁₇, X₁₈, X₁₉, X₃₄, X₃₉, and X₄₀ vary among particles of the plurality, the at least two varied positions being in the first and second interaction loop of the Kunitz domain.
10. The library of claim 9 wherein the display protein comprises the gene III coat protein stump.
11. The library of claim 9 wherein the phage genes comprise a gene that encodes a wild-type gene III coat protein.
12. The library of claim 9 wherein the library has a theoretical diversity of between 10³ and 10¹².
13. The library of claim 12 wherein at least amino acid positions 15, 16, 17, 18, 34, and 39 are varied.
14. The library of claim 12 wherein amino acid positions 11, 13, 15, 16, 17, 18, 19, 34, 39, and 40 are varied.
15. The library of claim 9 wherein the average number of the average number of copies of the Kunitz domain per phage particles of the plurality is less than 1.5.
16. A method of providing a nucleic acid encoding a Kunitz domain that interacts with a target, the method comprising:
   providing the library of claim 1;
   contacting phage particles form the library to a target; and
   recovering nucleic acid from particles that interact with the target.
17. The method of claim 16 wherein the target is immobilized, and the step of recovering comprises separating particles that interact with the target from particles that do not interact with the target.
18. A method of providing a nucleic acid encoding a Kunitz domain that interacts with a target, the method comprising:
   providing the library of claim 9;
   contacting phage particles form the library to a target; and
   recovering nucleic acid from particles that interact with the target.
19. The method of claim 18 wherein the target is a protease.
20. A phage vector comprising:
   (a) phage genes sufficient to produce an infectious phage particle and (b) a sequence encoding a display protein,
   the display protein comprising a functional domain of a minor coat protein and a Kunitz domain comprising a sequence that is at least 85% identical to MHSFCAFKADX₁₁GX₁₃CX₁₅X₁₆X₁₇X₁₈X₁₉RFFFNIFTRQCEEFX₃₄YGGCX₃₉X₄₀ NQNRFESLEECKKMCTRDGA, at positions other than X;
   X₁₁ is one of: A, D, E, F, G, H, I, K, L, M, N, P, Q, R, S, T, V, W, Y;
   X₁₃ is one of: A, D, E, F, G, H, I, K, L, M, N, P, Q, R, S, T, V, W, Y;
   X₁₅ is one of: A, D, E, F, G, H, I, K, L, M, N, Q, R, S, T, V, W, Y;
   X₁₆ is one of: A, G, E, D, H, T;
   X₁₇ is one of: A, D, E, F, G, H, I, K, L, M, N, Q, R, S, T, V, W, Y;
   X₁₈ is one of. A, D, E, F, G, H, I, K, L, M, N, Q, R, S, T, V, W, Y;
   X₁₉ is one of: A, D, E, F, G, H, I, K, L, M, N, P, Q, R, S, T, V, W, Y;
   X₃₄ is one of: A, C, D, E, F, G, H, I, K, L, M, N, P, Q, R, S, T, V, W, Y;
   X₃₉ is one of: A, C, D, E, F, G, H, I, K, L, M, N, P, Q, R, S, T, V, W, Y; and
   X₄₀ is one of: G, A, wherein the phage genes comprise a gene encoding the minor coat protein that is not fused to a heterologous sequence greater than five amino acids in length.

## Claims

1. A population of host cells comprising a plurality of filamentous phage particles, each phage particle of the plurality including
(i) a display protein that comprises a Kunitz domain, and
(ii) a nucleic acid comprising (a) phage genes sufficient to produce an infectious phage particle and (b) a sequence encoding the display protein, wherein the Kunitz domain includes at least one amino acid position in each of two interaction loops, the positions in the respective loops being varied among the particles of the plurality, and wherein the average number of copies of the Kunitz domain per phage particles of the plurality is less than 2.0. [[*page 8, lines 6-12; page 27, lines 27-29; page 30, lines 21-32; page 32, lines 14-17]]*

2. The host cells of claim 1, wherein the Kunitz domain is at least 85% identical to a human Kunitz domain at amino acid positions that are not varied.

3. The host cells of claim 1, wherein between 5 and 12 amino acid positions are varied among the particles of the plurality.

4. The host cells of claim 3, wherein at least amino acid positions corresponding to amino acid positions 16, 17, 18, 19, 34, and 39 of human LACI-K1 are varied among the particles of the plurality.

5. The host cells of claim 4, wherein at least amino acid positions corresponding to amino acid positions 11, 13, 15, 16, 17, 18, 19, 34, and 39 of human LACI-K1 are varied among the particles of the plurality.

6. The host cells of claim 3, wherein only amino acid positions corresponding to amino acid positions 11, 13, 15,16, 17, 18, 19, 34, 39, and 40 of human LACI-K1 are varied among the particles of the plurality.

7. The host cells of claim 1, wherein the amino acid position corresponding to amino acid position 40 is G or A, or is varied between G and A.

8. The host cells of claim 1, wherein the display protein comprises a functional domain of a minor coat protein fused to the Kunitz domain, and the phage genes comprise a gene that encodes the minor coat protein that is (i) not fused to a non-viral amino acid sequence of at least five amino acids or (ii) not fused to a varied amino acid sequence.

9. The host cells of claim 1, wherein the display protein further comprises at least a portion of the gene III phage coat protein.

10. The host cells of claim 1, wherein the Kunitz domain comprises a sequence that is at least 85% identical to
MHSFCAFKADX₁₁GX₁₃CX₁₅X₁₆X₁₇X₁₈X₁₉RFFFNIFTRQCEEFX₃₄YGGCX₃₉X₄₀ NQNRFESLEECKKMCTRDGA, at positions other than X;
X₁₁ is one of: A, D, E, F, G, H, I, K, L, M, N, P, Q, R, S, T, V, W, Y;
X₁₃ is one of: A, D, E, F, G, H, I, K, L, M, N, P, Q, R, S, T, V, W, Y;
X₁₅ is one of: A, D, E, F, G, H, I, K, L, M, N, Q, R, S, T, V, W, Y;
X₁₆ is one of: A, G, E, D, H, T;
X₁₇ is one of. A, D, E, F, G, H, I, K, L, M, N, Q, R, S, T, V, W, Y;
X₁₈ is one of: A, D, E, F, G, H, I, K, L, M, N, Q, R, S, T, V, W, Y;
X₁₉ is one of: A, D, E, F, G, H, I, K, L, M, N, P, Q, R, S, T, V, W, Y;
X₃₄ is one of: A, C, D, E, F, G, H, I, K, L, M, N, P, Q, R, S, T, V, W, Y;
X₃₉ is one of: A, C, D, E, F, G, H, I, K, L, M, N, P, Q, R, S, T, V, W, Y; and
X₄₀ is one of: G, A, and
at least two of X₁₁, X₁₃, X₁₅, X₁₆, X₁₇, X₁₈, X₁₉, X₃₄, X₃₉, and X₄₀ vary among particles of the plurality, the at least two varied positions being in the first and second interaction loop of the Kunitz domain.

11. The host cells of claim 1, wherein the display protein comprises the gene III coat protein stump.

12. The host cells of claim 1, wherein the phage genes comprise a gene that encodes a wild-type gene III coat protein.

13. The host cells of claim 1, wherein the plurality of filamentous phage has a theoretical diversity of between 10³ and 10¹².

14. The host cells of claim 13, wherein at least amino acid positions 15, 16, 17, 18, 34, and 39 are varied.

15. A library comprising a plurality of filamentous phage particles, each phage particle of the plurality including
(i) a display protein that comprises a Kunitz domain,
(ii) a nucleic acid comprising (a) phage genes sufficient to produce an infectious phage particle and (b) a sequence encoding the display protein, wherein the Kunitz domain includes at least varied one amino acid position in each of two interaction loops, the positions in the respective loops being varied among the particles of the plurality, and wherein the average number of copies of the Kunitz domain per phage particles of the plurality is less than 2.0.
